# EUROPEAN PATENT SPECIFICATION

(11) **EP 3 250 142 B1**
(45) Date of publication and mention of the grant of the patent: **02.09.2026**
(21) Application number: 16744266.4
(22) Date of filing: 01.02.2016
(51) Int. Cl.: A61B 18/18, A61B 18/14, A61B 18/00, A61B 34/20, A61B 10/02

(54) **SYSTEM FOR RADIO-FREQUENCY ELECTRICAL MEMBRANE BREAKDOWN FOR THE TREATMENT OF TISSUES**
SYSTEM FÜR ELEKTRISCHE MEMBRANAUFLÖSUNG MITTELS HOCHFREQUENZ ZUR BEHANDLUNG VON GEWEBE
SYSTÈME POUR RUPTURE DE MEMBRANE ÉLECTRIQUE PAR RADIO-FRÉQUENCE POUR LE TRAITEMENT DE TISSUS

(30) Priority: 30.01.2015 US 201562109965 P; 02.02.2015 US 201562110646 P; 02.02.2015 US 201562110674 P; 02.02.2015 US 201562110733 P; 02.02.2015 US 201562110702 P; 04.02.2015 US 201562111870 P
(43) Date of publication of application: 06.12.2017
(73) Proprietor: Rfemb Holdings LLC, North Branford, CT 06471 (US)
(72) Inventor: ONIK, Gary, M., Ft. Lauderdale, FL 33301 (US); MIESSAU, James, A., Branford, CT 06405 (US); BOSTWICK, David, G., Orlando, FL 32837 (US)
(74) Representative: Barker Brettell LLP
(86) International application number: PCT/US2016/015944
(87) International publication number: WO 2016/123608

(56) References cited:
- WO-A1-2015/085162
- US-A1- 2003 055 471
- US-A1- 2003 163 040
- US-A1- 2003 163 040
- US-A1- 2009 088 648
- US-A1- 2010 049 031
- US-A1- 2010 049 031
- US-A1- 2011 015 630
- US-A1- 2011 015 630
- US-A1- 2011 202 053
- US-A1- 2012 109 122
- US-A1- 2014 356 397

## Description

### BACKGROUND OF THE INVENTION

### 1. Field of the invention

The present invention relates generally to medical devices, and more particularly, to a device utilizing radio frequency electrical membrane breakdown ("RFEMB") to treat prostate cancer, unresectable pancreatic cancer, tumors of the breast or soft tissue, melanoma, ductal carcinoma, neoplasia, or intra and extra luminal abnormal tissue by ablating cancerous tissue using applied electric fields, and to use the immediate tumor necrosis caused by RFEMB to enhance the patient's immune response to the ablated cells. Known ablation devices are disclosed in WO 2015/085162 (document falling under Article 54(3) EPC), and in US2011/0015630.

### 2. Background of the Invention

Cancer is not one single disease but rather a group of diseases with common characteristics that often result in sustained cell proliferation, reduced or delayed cell mortality, cooption of bodily angiogenesis and metabolic processes and evasion of bodily immune response which results in undesirable soft tissue growths called neoplasms or, more commonly, tumors. Removal or destruction of this aberrant tissue is a goal of many cancer treatment methods and modalities. Surgical tumor excision is one method of accomplishing this goal. Tissue ablation is another, minimally invasive method of destroying undesirable tissue in the body, and has been generally divided into thermal and non-thermal ablation technologies. Thermal ablation encompasses both the addition and removal of heat to destroy undesirable cells. Cryoablation is a well established thermal ablation technique that kills cells by freezing of the extracellular compartment resulting in cell dehydration beginning at -15 C and by intracellular ice formation causing membrane rupture occurring at colder temperatures. Because cryoablative techniques can rupture the cell membrane without denaturing cell proteins under certain conditions, such techniques have the additional ability to stimulate an antitumor immune response in the patient.

Heat based techniques are also well established for ablation of both cancerous and non-cancerous tissues and include radio-frequency (RF) thermal, microwave and high intensity focused ultrasound ablation which raise localized tissue temperatures well above the body's normal 37° C. These methods use various techniques to apply energy to the target cells to raise interstitial temperature. For example, RF thermal ablation uses a high frequency electric field to induce vibrations in the cell membrane that are converted to heat by friction. Cell death occurs in as little as thirty (30) seconds once the cell temperature reaches 50°C and increases as the temperature rises. At 60°C cell death is instantaneous. If the intracellular temperature rises to between about 60 and 95°C, the mechanisms involved in cell death include cellular desiccation and protein coagulation. When the intracellular temperature reaches 100°C, cellular vaporization occurs as intracellular water boils to steam. In the context of tissue ablation, cell temperatures not exceeding 50°C are not considered clinically significant Because cellular proteins are denatured by the heat of thermal ablation techniques, they are not available to stimulate a specific immune response as they may be with cryoablation. Both heat based and cryoablation techniques suffer from the drawback that they have little or no ability to spare normal structures in the treatment zone and so can be contraindicated based on tumor location or lead to complications from collateral injury.

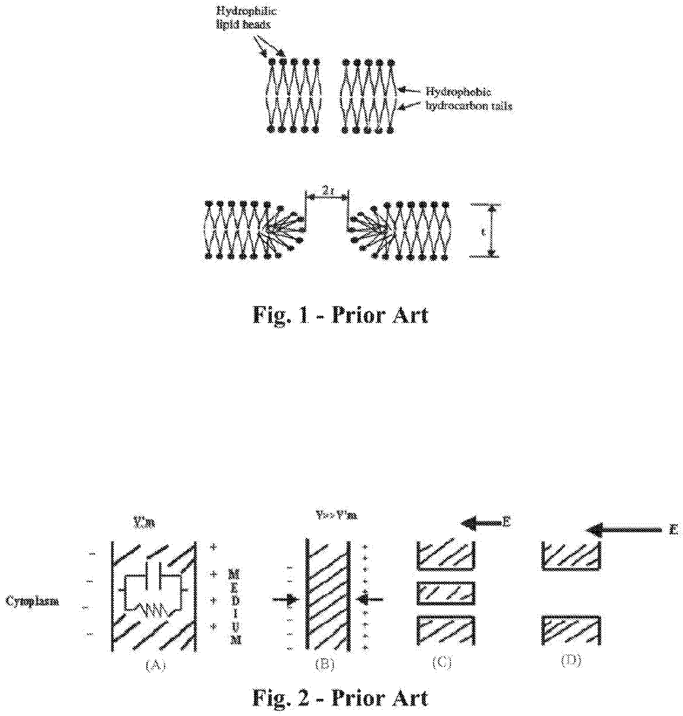

Non thermal ablation techniques include electrochemotherapy and irreversible electroporation (IRE), which although quite distinct from one another, each rely on the phenomenon of electroporation. With reference to FIG. 1, electroporation refers to the fact that the plasma membrane of a cell exposed to high voltage pulsed electric fields within certain parameters, becomes temporarily permeable due to destabilisation of the lipid bilayer and the formation of pores P. The cell plasma membrane consists of a lipid bilayer with a thickness t of approximately 5 nm. With reference to FIG. 2(A), the membrane acts as a nonconducting, dielectric harrier forming, in essence, a capacitor. Physiological conditions produce a natural electric potential difference due to charge separation across the membrane between the inside and outside of the cell even in the absence of an applied electric field. This resting transmembrane voltage potential (V'm) ranges from 40mv tor adipose cells to 85mv concentration, among other things.

With continued reference to FIGS. 2(B)-2(D), exposure of a cell to an externally applied electric field E induces an additional voltage V across the membrane as long as the external field is present. The induced transmembrane voltage is proportional to the strength of the external electric field and the radius of the cell. Formation, of transmembrane pores P in the membrane occurs if the cumulative resting and applied transmembrane potential exceeds the threshold voltage which may typically be between 200 mV and 1 V. Poration of the membrane is reversible if the transmembrane potential does not exceed the critical value such that the pore area is small in relation to the total membrane surface. In such reversible electroporation, the cell membrane recovers after the applied field is removed and the cell remains viable. Above a critical transmembrane potential and with longer exposure times, poration becomes irreversible leading to eventual cell death due an influx of extracellular ions resulting in loss of homeostasis and subsequent apoptosis. Pathology after irreversible electroporation of a cell does not show structural or cellular changes until 24 hours after field exposure except in certain very limited tissue types. However, in all cases the mechanism of cellular destruction and death by IRE is apoptotic which requires considerable time to pass and is not visible pathologically in a time frame to be clinically useful in determining the efficacy of IRE treatment, which is an important clinical drawback to the method.

Developed in the early 1990's, electrochemotherapy combines the physical effect of reversible cell membrane poration with administration of chemotherapy drugs such as cisplatin and bleomycin. By temporarily increasing the cell membrane permeability, uptake of non-permeant or poorly permeant chemotherapeutic drugs is greatly enhanced. After the electric field is discontinued, the pores close and the drug molecules are retained inside the target cells without significant damage to the exposed cells. This approach to chemotherapy grew out of earlier research developing electroporation as a technique for transection of genes and DNA molecules for therapeutic effect. In this context irreversible electroporation leading to cell death was viewed as a failure in as much as the treated cells did not survive to realize the modification as intended.

IRE as an ablation method grew out of the realization that the "failure" to achieve reversible electroporation could be utilized to selectively kill undesired tissue. IRE effectively kills a predictable treatment area without the drawbacks of thermal ablation methods that destroy adjacent vascular and collagen structures. During a typical IRE treatment, one to three pairs of electrodes are placed in or around the tumor. Electrical pulses carefully chosen to induce an electrical field strength above the critical transmembrane potential are delivered in groups of ten, usually for nine cycles. Each ten-pulse cycle takes about one second, and the electrodes pause briefly before starting the next cycle. As described in U.S. Patent No. 8,048,06.7 to Rubinsky, et. al and U.S. Patent Application No. 13/332,133 to Arena, et al., the field strength and pulse characteristics are chosen to provide the necessary field strength for IRE but without inducing thermal effects as with R.F thermal ablation.

However, the DC pulses used in currently available IRE methods and devices have characteristics that can limit their use or add risks for the patient because current methods and devices create severe muscle contraction during treatment. This is a significant disadvantage because it requires that a patient be placed and supported under general anesthesia with neuromuscular blockade in order for the procedure to be carried out, and this carries with it additional substantial inherent patient risks and costs. Moreover, since even relatively small muscular contractions can disrupt the proper placement of IRE electrodes, the efficacy of each additional pulse train used in a therapy regimen may be compromised without even being noticed during the treatment session. In addition, the high voltage DC pulses used by IRE may cause sparks to occur at the junction of the electrode and its insulation. These sparks can be of such an intensity as to cause a physical disruption of tissue leading to local complications.

Cancer cells produce antigens, which the immune system can use to identify and destroy them. These antigens are recognized by dendritic cells, which present the antigens to Cytotoxic T lymphocytes (CTLs) in the lymph nodes. The CTLs can then recognize the cancer cells by those antigens and destroy them. However, dendritic cells present cancer antigens to CTLs along with an inhibitory signal, which binds to a receptor, cytotoxic T lymphocyte-associated antigen 4 (CTLA-4). on the CTL and turns off the cytotoxic reaction, interrupting the destruction of the cancer cells and allowing the cancer cells to survive. See Antoni Ribas, "Tumor immunotherapy directed at PD-1", New England Journal of Medicine 366 (26): 2517-9 (28 June 2012).

Approaches to modulate this tumor immune response, in general, are now available and have been shown to have positive effects, improving patient survival in certain tumor types. One of these approaches utilizes Sipuleucel-T (Provenge), which uses autologous patient dendritic cells activated with PAP infused back into the patient, has been shown in multiple studies to improve survival in hormone resistant prostate cancer by an average of approximately 4 months. Sipuleucel-T showed overall survival (OS) benefit to patients in three double-blind randomized phase III clinical trials. A recent trial of a similar approach for Pancreatic cancer using Algenpantucel-L in a phase two study was demonstrated to be safe while prolonging survival, beyond that projected by historical controls. See Hardacre, J, Mulcahy, M., Small, W., Talanionti, M., Obel, J. and Lima, R., "Addition of algenpantucel-L immunotherapy to standard of care (SOC) adjuvant therapy for pancreatic cancer", J Clin Oncol 2012; 30(Suppl); abstract 4049 (observed survival of 86% compared to predicted 1 year of 55-63%).

Ipilimumab, marketed as Yervoy, is a human monoclonal antibody and works by blocking the CTLA-4 inhibitory signal allowing the CTLs to destroy the cancer cells. CTLA-4 (also known as CD 152) is expressed on the surface of T cells along with the co-stimulatory receptor CD28. In contrast to CD28, which activates T cells when bound to ligands of antigen presenting cells (APCs), CTLA-4 interferes with IL-2 production, IL-2 receptor expression, interrupts cell cycle progression of activated T cells, and antagonizes T cell activation. Inhibition of CTLA-4 receptors using Ipilimumab reportedly resulted in increased activity of T cells and led to tumor regression. is a drug approved for the treatment of metastatic melanoma that works on a different aspect of the immune system. Studies have shown Ipilimumab to improve survival in patients with metastatic melanoma, but Ipilimumab alone has been shown to be unsuccessful as a single agent in pancreatic cancer. See Royal RE, Levy C, Turner K et at., "Phase 2 trial of single agent Ipilimumab (anti-CTLA-4) for locally advanced or metastatic pancreatic adenocarcinoma", J Immunother. 2010 Oct; 33(8):828-33.

A third class of drug with immunomodulatory effects is Tasquinimod. Tasquinimod is a novel small molecule that targets the tumor microenvironment by binding to S100A9 and modulating regulatory myeloid cell functions, exerting immunomodulatory, anti-angiogenic and anti-metastatic properties. Tasquinimod may also suppress the tumor hypoxic response, contributing to its effect on the tumor microenvironment. It has been shown to have significant clinical effects in castrate resistant prostate cancer.

Because cells ablated by IRE methods undergo apoptotic death without membrane rupture, their ability to induce a supplemental immune response as observed with cryoablation is impaired. When used as the sole ablative tool in a treatment protocol, IRE's inability to induce a supplemental immune response is a substantial limitation to its therapeutic benefit for patients.

On the other hand, cryoablation is limited by significant clinical disadvantages arising from the extreme cold and its capacity to destroy nearby critical healthy structures. Other limitations of cryosurgery include: (1) expense of equipment; (2) time consuming creation of the cryolesion; (3) logistical problems associated with cryogen handling; (4) inability to immediately biopsy the cryolesion and assess the success of the procedure; (5) slow resolution of the cryolesion leading to questions of treatment adequacy and patient anxiety; and (6) the potential for destruction of the structural network of tissue leading to tissue sloughing and perforation.

### Pancreatic Cancer

While pancreatic cancer survival rates have been improving from decade to decade, the disease is still considered largely incurable. According to the American Cancer Society, for all stages of pancreatic cancer combined, the one-year relative survival rate is 20%, and the five-year rate is 6%. These low survival rates are attributable to the fact that fewer than 20% of patients' tumors are confined to the pancreas at the time of diagnosis and in most cases, the malignancy has already progressed to the point where surgical removal is impossible. In those cases where resection can be performed, the average survival rate is 18 to 20 months. The overall five-year survival rate is about 10%, although this can rise as high as 20% to 25% if the tumor is removed completely and when cancer has not spread to lymph nodes. Therefore, any major advance in pancreatic cancer therapy must, take into account the fact that most cancers are locally unresectable and that distant disease either gross or microscopic is usually present when a patient is already diagnosed.

Unfortunately, cryosurgical ablation of pancreatic cancer to harness its demonstrated immunologic effect has not been feasible due to cryosurgery's propensity to destroy surrounding structures such as bowel and bile ducts causing unacceptable complications. IRE is an ablation modality that spares structures such as bowel, ducts and nerves and has therefore been safely employed in the treatment of unresectable pancreatic cancer. However, the documented limitations of IRE, including severe muscle contraction and sparking during treatment, and apoptotic method of cell destructive have severely hampered IRE's usefulness as a treatment modality for pancreatic cancer. While data suggests that the use IRE to treat, unresectable pancreatic cancer can improve overall survival in this group of patients, the overwhelming majority of pancreatic patients die from distant metastatic disease due to IRE's inability to induce an immunologic response with treatment.

### Prostate Cancer

For the treatment of prostate cancer, focal therapies such as focal cryoablation are gaining acceptance among physicians as a middle ground, between "watchful waiting" with no immediate therapeutic action and whole gland/tumor therapies, such as radical prostatectomy or radiation therapy, which are often, associated with significant morbidities, particularly in the setting of high risk prostate cancer, and which often are not even clinically useful in cases of recurrent disease. At the present time, all methods for carrying out focal therapy involve technologies that require full operating room capabilities, or imaging capabilities such as MM, both of which are very expensive and in relatively limited supply. As an additional downside, high risk prostate cancers, such as cancers involving High Gleason score, high Prostate-Specific Antigen (PSA), or high grade prostate cancer, have high recurrence rates, approaching 40% according to some studies, both locally and distantly when treated with conventional therapies such as radical prostatectomy and radiation therapy.

Meanwhile, very important clinical advantages have been demonstrated by the instant inventors in cases high risk prostate cancer patients treated with focal cryoablation. Their studies have demonstrated a dramatic improvement in overall long term survival, which they attribute to the immunological effects of focal cryoablation. This is also the case for patients who have failed a primary treatment such as radiation therapy or radical prostatectomy and who have locally recurrent disease that is treated using focal cryoablation. While these advantages are considerable, successful focal cryoablation is greatly dependent on the skill of the physician applying it and the individual techniques and methods they use. Prior art methods require substantial time and particular treatment procedures and repeated freeze and thaw cycles around very specific parameters in order to be effective and avoid damage to nearby structures.

### Breast Cancer

Breast cancer is the most common type of malignancy occurring in women worldwide. Despite medical advances of recent years, standard therapeutic responses to breast cancer leave much to be desired. Physically, standard therapies are typically painful, debilitating., and often mutilating as well. Psychologically, fear of disfigurement and loss of femininity resulting from treatment add to the psychological burden associated with cancer diagnoses and cancer treatments of any sort. Thus, there is a widely felt need for, and it would be highly advantageous to have, a therapeutic approach to benign and malignant breast tumors which speeds, shortens and simplifies clinical treatment of breast tumors tentatively diagnosed as benign, and which speeds, shortens, and simplifies pre-operative treatment of tumors thought to be malignant.

In the case of benign tumors of the breast (e.g., fibroadenomas) and of certain small malignant tumors, ablation of the pathological tissue material is the therapy. In the case of large malignant tumors, downsizing of the tumor may be an important preliminary step in a multi-step therapeutic process. With respect, to benign tumors, classical excision therapy, removal of a tumor by simply cutting out the offending material, is often not an optimal form of treatment. The preparation, process, and aftermath of classical excision surgery lead to great anxiety and psychological stress in many women. The therapy is frightening, and the recovery is painful. Moreover, surgical excision is likely to cause scarring or other minor or major disfigurement. Breast deformation may result. Further, seen from a generalized social point of view, classical excision surgery as treatment for benign breast tumors is a relatively costly process, generally requiring hospitalization. Thus there is a widely felt need for, and it would be highly advantageous to have, an apparatus for therapeutic treatment of benign breast tumors and of small malignant tumors, which apparatus is minimally invasive and are less traumatic than surgical excision, which yield superior cosmetic results when compared to classical excision therapy, and which can be performed as an outpatient procedure.

Not all breast tumors, of course, are benign. Many breast tumors are malignant, and despite efforts at early detection, many malignant tumors are diagnosed when they are in an advanced stage of development.

Early stage tumors have a better prognosis than advanced stage tumors, and are simpler to treat. Advanced stage tumors, typically of larger size and often having lymph node involvement and/or metastases, are considerably harder to treat successfully. Advanced stage tumors require more extensive and complex therapies, and. typically do require classical excision surgery.

**In** addition, there are some tumors such as those that invade the chest wall that cannot be surgically removed. An ablation modality that, can ablate these tumors gaining local control would be a very desirable result.

Breast cancer is the second leading cause of mortality among women. Breast cancer-related mortality is almost invariably due to metastasis. Between 25% and 50% of patients diagnosed with breast cancer will eventually develop deadly metastases, often decades after the diagnosis and removal of the primary tumor. The prognosis for patients with metastatic breast cancer (MBC) is generally unfavorable, with an average 5-year survival rate of only about 25%. The therapeutic alternatives for MBC are mainly based on the systemic administration of cytotoxic chemotherapeutic agents; the long-term impact on survival, however, is only twenty (20) months and depends heavily on the nature of the metastases and tumor biology.

While substantial advances have been made in the treatment of localized malignancies, metastatic disease still lacks effective treatment and remains the primary cause, of mortality due to cancer, including breast cancer. Thus, to increase the survival of cancer patients, it is necessary to effectively improve the prevention or treatment of metastasis. Therefore a treatment that allows for efficient, low toxicity adjuvant, therapy, that also treats the local, tumor, and that could therefore prevent future distant metastasis, would be a significant advance. It would also be greatly advantageous if this treatment could be applied to patients that already have metastatic disease.

### Melanoma and Other Skin Malignancies

Melanoma, a type of skin cancer caused due to uncontrolled proliferation of epidermal melanocytes, is notorious for its rapid progression, metastasis, and poor prognosis; it is responsible for over 80 % of all deaths from skin cancer. A recent study from the USA reports it as the fifth most frequent cancer in men and seventh most frequent cancer in women.

Despite medical advances of recent years, standard therapeutic approaches to melanoma both for the primary tumor and metastatic disease leave much to be desired. Physically, standard therapies such as surgery for the primary tumor are typically painful, debilitating, and often mutilating as well. This is particularly true based on the location of the primary tumor, which can literally be located on any skin surface of the body including the face and genitals. Fear of disfigurement, resulting from treatment adds to the psychological burden associated with melanoma diagnosis and cancer treatments of any sort.

Patients with advanced melanoma and unresectable tumors have very poor prognosis, and less than 20% of patients with metastatic melanoma survive for five years. Metastatic melanoma is well known for its resistance to conventional radio and chemotherapy, and until recently, dacarbazine, a DMA alkylating agent, and high dose interleukin-2 (HD IL-2) were the only approved treatment options available for patients.

Since 2011, there have been considerable advances in melanoma treatment with the approval of inhibitors of the BRAF gene (mutations of which has been shown to promote certain cancers) and mitogen-activated protein kinase (MEK), such as Vemurafenib and Dabrafenib, specific inhibitors of BrafV600E (BRAF harboring a point mutation resulting from a substitution of valine at ammo-acid 600 with glutamine), such as Trametinib, a specific inhibitor of MEK and Ipilimumab, a monoclonal antibody against CTLA-4. The success of Ipilimumab in stabilizing the disease and increasing the overall survival has particularly interested clinicians in using immunotherapy as an option for melanoma management.

Based on a randomized, double blind, double dummy clinical trial which reported survival benefits in Ipilimumab-treated melanoma patients as compared to those treated with a peptide-based vaccine, and showed an overall survival 10.1 vs 6.4 months, Ipilimumab was approved in 2011 for the treatment of unresectable melanoma. The patients enrolled in the study were followed for up to fifty-five months, and drug resistance was not reported in responding patients.

Ipilimumab is recommended as a first-line therapy in BRAF(-) unresectable stage IV melanoma patients with 'poor performance status' and as second-line therapy in patients with 'good performance status' irrespective of BRAF mutation. The panel suggests reserving BRAF and MEK inhibitors in BRAF(+) patients with 'good performance status' for later stages and recommends using them in patients who do not respond to IL-2 and Ipilimumab treatment. The major limitation with Ipilimumab treatment is poor response rate in patients, as seen by only 2-14% of both partially and completely responding patients and nearly 50-60% of unresponsive patients to Ipilimumab treatment. Another major limitation of Ipilimumab is that many patients suffer from severe autoimmune side effects, which can limit the duration of therapy and can cause death (approximately 25% of patients will need to stop therapy due to these side effects). Several strategies, like combining Ipilimumab with human monoclonal anti-PD1 or anti-phosphatidylserine antibodies (nivolumab and bavituximab, respectively) or with BRAF inhibitors (Dabrafenib) or radiotherapy, have been proposed to increase the response rate, and these combination therapies are currently in various stages of clinical trials.

Thus, there is a widely felt need for, and it would be highly advantageous to have, a therapeutic approach to melanoma which aids in the treatment of the primary tumor, particularly those in difficult locations that could lead to disfigurement, In addition, it would be advantageous to somehow improve the survival of later stage high risk melanoma patients, either in an adjuvant fashion to be used at the time of primary treatment to prevent future metastatic disease, or in combination with other immunotherapies such as Ipilimumab to improve the responses rate in patients already suffering with metastatic disease.

For small melanoma tumors, surgical removal of pathological tissue material is the therapy. With respect to small melanomas, classical excision therapy, removal of a tumor by simply cutting out the offending material, is often not an optimal form of treatment. The preparation, process, and aftermath of classical excision surgery lead to great anxiety and psychological stress in many patients. The therapy is frightening, and the recovery is painful. Moreover, surgical excision is likely to cause scarring or other minor or major disfigurement. Skin deformation may result or large skin grafts needed. Further, seen from a generalized social point of view, classical excision surgery as treatment for melanoma is a relatively costly process. Thus there is a widely felt need for, and it would be highly advantageous to have, an apparatus for therapeutic treatment of small malignant melanomas, which apparatus is minimally invasive and are less traumatic than surgical excision, which yield superior cosmetic results when compared to classical excision therapy, and which can he performed as an outpatient procedure.

While substantial advances have been made in the treatment of localized malignancies, metastatic disease still lacks effective treatment and remains the primary cause of mortality due to cancer, including melanoma. Thus, to increase the survival of melanoma patients, it is necessary to effectively improve the prevention or treatment of metastasis as well.

### Sarcoma and Soft Tissue Tumors

A sarcoma is a type of cancer that develops from certain tissues, like bone or muscle. There are 2 main types of sarcoma: bone sarcomas and soft tissue sarcomas. Soft tissue sarcomas can. develop from soft tissues like fat, muscle, nerves, fibrous tissues, blood vessels, or deep skin tissues. They can be found in any part of the body. Most of them develop in the arms or legs. They can also be found in the trunk, head and neck area, internal organs, and the area in back, of the abdominal cavity (known as the retroperitoneum). **In** addition, undesirable soft tissue tumors may comprise one or more tumors or cancer of the brain, central nervous system or spinal cord. Sarcomas axe not common tumors.

There are many types of soft tissue tumors, and not all of them are cancerous. When a tumor is not cancerous, it is called benign. When the term sarcoma is part of the name of a disease, it means the tumor is malignant (cancer). There are about fifty different types of soft tissue sarcomas and their benign counterpart; the following is a non-exhaustive list.
(1) Fat tissue tumors:
   (a) Benign fat tissue tumors: Lipomas are benign tumors of fat tissue. They are the most common benign soft tissue tumor. Most are found just under the skin, but they can develop anywhere in the body. Lipoblastomas are benign fat tumors that occur in infants and young children. Hibernomas, like lipomas, are also benign fat tissue tumors. They are much less common than lipomas.
   (b) Cancerous fat tissue tumors: Liposarcomas are malignant tumors of fat tissue. They can develop anywhere in the body, but they most often develop in the thigh, behind the knee, and inside the back of the abdomen. They occur mostly in adults between 50 and 65 years old.
(2) Muscle tissue tumors:
   (a) Benign muscle tumors: Leiomyomas are benign tumors of smooth muscle. Leiomyomas can start from the walls of blood vessels, so they can develop almost anywhere in the body. They can be found in both men and women, but the most, common place to find a leiomyoma is in the walls of the uterus. They are often called fibroids. Rhabdomyomas are rare benign tumors of skeletal muscle.
   (b) Malignant muscle tumors: Leiomyosarcomas are malignant tumors of smooth muscle. Like leiomyomas, they cars grow almost anywhere in the body. They are most often found in the retroperitoneum (area in back of the abdominal cavity), the internal organs, and blood vessels. These tumors are less often found in the deep soft tissues of the legs or arms. They tend to occur in adults, particularly the elderly. Rhabdomyosarcomas are malignant tumors of skeletal muscle. These tumors commonly grow in the arms or legs, but they can also begin in the head and neck area and in reproductive and urinary organs like the vagina or bladder. Children are affected much more often than adults.
(3) Peripheral nerve tissue tumors:
   (a) Benign nerve tumors: Neurofibromas, schwannomas (neurilemmomas), and neuromas are all benign minors of nerves. These tumors can occur almost anywhere in the body. Neurofibromas are very common in people with an inherited condition called neurofibromatosis (also called von Recklinghausen disease). Sometimes neurofibromas of very large nerves (like those in the upper arms or neck) can become malignant.
   (b) Malignant nerve tumors: Neurofibrosarcomas, malignant schwannomas, and neurogenic sarcomas are malignant tumors of the cells that surround a nerve. These are also called malignant peripheral nerve sheath tumors.
(4) Gastrointestinal stromal tumor (GIST) is a type of sarcoma that develops in the digestive tract. It starts in the cells that control the muscles lining the stomach and intestine. These muscles propel food through the digestive tract.
(5) Joint tissue tumors:
   (a) Benign joint tumors: Nodular tenosynovitis is a benign tumor of joint tissue, ft is most common in the hands and is more common in women than in men.
   (b) Malignant joint tumors: Synovial sarcoma is a malignant tumor of the tissue around joints. The most common locations are the knee and ankle. Other sites are the shoulder and hip. This tumor is more common in children and young adults, but it can occur in older people.
(6) Fibrous tissue tumors:
   (a) Benign fibrous tumors: These include: Fibromas, Elastofibromas, Superficial fibromatosis, and Fibrous histiocytomas.
   (b) Intermediate fibrous tumors; Fibromatosis is the name given to fibrous tissue tumor with features in between fibrosarcoma and benign tumors such as fibromas and superficial fibromatosis. They tend to grow slowly but, often, steadily. These tumors are also called desmoid tumors, as well as the more scientific name musculoaponeurotic fibromatosis. They do not spread to distant sites, but they do cause problems by growing into nearby tissues. They can sometimes be fatal Some doctors consider them a type of low-grade fibrosarcoma, bat others believe they are a unique type of fibrous tissue tumors. Certain hormones, particularly estrogen, make some desmoid tumors grow. Anti-estrogen drugs are sometimes useful in treating desmoids that cannot be completely removed by surgery. Dermatofibrosarcoma protuberans is a slow-growing cancer of the fibrous tissue beneath the skin, usually in the trunk or limbs. It grows into nearby tissues but rarely spreads to distant sites.
   (c) Malignant fibrous tumors: Fibrosarcoma is cancer of fibrous tissue. It usually affects the legs, arms, or trunk. It is most common in people between the ages of 20 and 60, but can occur at any age, even in infancy.

Despite medical advances of recent years, standard therapeutic responses to soft tissue tumors leave much to be desired. Physically, standard therapies are typically painful, debilitating, and often mutilating as well. As in the case of melanoma, fear of disfigurement resulting from treatment adds to the psychological burden associated with cancer diagnoses and cancer treatments of any sort.

Thus, there is a widely felt need for, and it would be highly advantageous to have, a therapeutic approach to benign and malignant soft tissue tumors which speeds, shortens and simplifies clinical treatment of soft tissue tumors tentatively diagnosed as benign, and which speeds, shortens, and simplifies treatment of tumors thought, to be malignant.

Also as with melanomas, surgical excision of the pathological tissue material is the therapy for sarcoma and soft tissue tumors, but classical excision, therapy suffers from the same drawbacks discussed above with reference to melanomas. Thus there is a widely felt need for, and it would be highly advantageous to have, an apparatus for therapeutic treatment of benign soft tissue minors and of small malignant tumors, which apparatus is minimally invasive and are less traumatic than surgical excision, which yield superior cosmetic results when compared to classical excision therapy, and which can be performed as an outpatient procedure.

**In** addition, there are some soft tissue tumors such as those that invade the retroperitoneum that cannot be surgically removed even when tumor downsizing is attempted. An ablation modality that can ablate these tumors gaining local control would be a very desirable result.

Soft tissue cancer-related mortality is almost invariably due to metastasis. While substantial advances have been made in the treatment of localized malignancies, metastatic disease still lacks effective treatment and remains the primary cause of mortality due to cancer, including soft tissue cancer. Thus, to increase the survival of cancer patients, it is necessary to effectively improve the prevention or treatment of metastasis.

### Ductal Carcinoma, Neoplasia and Intra- and Extra-Luminal Abnormal Tissue

The use of image and endoscopically-guided catheters has revolutionized the treatment of many diseases. **In** the treatment of neoplasia, however, while tumor ablation has been successfully used with invasive needle type probes, the use of catheter based ablation has been limited due to the destructive thermal effects of available treatment modalities on the wall of critical anatomical structures such as ducts, bowel, vessels and urethra leading to complications such as stricture and perforation.

In addition, IRE is not an ideal ablation modality to be conducted from an intraluminal location from within bowel, duets, vessels or urethra, guided by either endoscopic means or other imaging guidance due to the drawbacks of the method as described above, including the propensity for IRE to cause muscular contractions and sparking at the treatment site.

Thus, the ability to create and monitor an ablative neoplasia destruction intraluminally through methods that do not have the inherent limitations of IRE or thermal cryoablation would be an advance. An ablation system that does not need neuromuscular blockade, spares tissue structure, does not cause potentially dangerous sparking and produces an immunologic response would provide an excellent means for treating neoplastic or other unwanted tissue if it could be delivered from an intraluminal location. Such a capability to treat from an. intraluminal location could be used to treat, for instance., bile duct carcinoma, carcinoma of the head of the pancreas, prostate neoplasia or BPH adjacent to the urethra, esophageal neoplasia and the pre-malignant lesion of Barrett's esophagus, to name a few.

For the treatment of all of the above conditions, what is needed is a minimally invasive tissue ablation technology that can avoid damaging healthy tissue while exposing cellular contents without denaturing such cellular contents so that they can trigger a clinically useful immune response.

In addition, an ablation system that can be accurately targeted at previously identified unwanted tissue, and that spares tissue structure outside of the focal treatment area, would be advantageous.

It would also be advantageous to provide a system for carrying out this treatment on an outpatient setting under local anesthesia, that does not require general anesthesia or a neuromuscular blockade, where appropriate, or alternatively on an inpatient intraoperative basis optionally utilizing an open, laparoscopic or robotic access to the treatment area.

### SUMMARY OF THE INVENTION

It is, therefore, an object of the present invention to provide a system for the treatment of prostate cancer, unresectable pancreatic cancer, tumors of the breast or soft tissue, melanoma, ductal carcinoma, neoplasia, or intra and extra luminal abnormal tissue using electrical pulses which causes immediate cell death through the mechanism of complete breakdown of the membrane of the target cells.

It is another object of the present invention to provide such a system that does not require the administration of general anesthesia or a neuromuscular blockade to the patient.

According to the invention there is provided a system for ablating undesirable soft tissue in a living subject as defined in claim 1. Further embodiments of the invention are defined by the dependent claims. Methods as such are not claimed. An embodiment of the present invention is an imaging, guidance, planning and treatment system integrated into a single unit or assembly of components that can be safely and effectively deployed to treat prostate cancer, unresectable pancreatic cancer, tumors of the breast or soft tissue, melanoma, ductal carcinoma, neoplasia, or intra and extra luminal abnormal tissue. The system utilizes the process of Radio-Frequency Electrical Membrane Breakdown ("EMB" or "RFEMB") to destroy the cellular membranes of unwanted or cancerous tissue without denaturing the intra-cellular contents of the cells comprising the tissue, thereby exposing tumor antigens and other intra-cellular components which can have an immunologic effect on local or distant cancerous tissue, with or without the addition of immunologic adjutant drugs.

The use of EMB to achieve focal tumor ablation with an enhanced immunologic effect on surrounding cancerous tissue is disclosed in U.S. Patent Application No. 14/4511,333 and International Patent Application. No. PCT/US14/68774.

EMB is the application of an external oscillating electric field to cause vibration and flexing of the cell membrane, which results in a dramatic and immediate mechanical tearing, disintegration and/or rupturing of the cell membrane. Unlike the IRE process, in which nano-pores are created in the cell membrane but through which little or no content of the cell is released, EMB completely tears open the cell membrane such that the entire contents of the cell are expelled into the extracellular fluid, and internal components of the cell membrane itself are exposed. EMB achieves this effect by applying specifically configured electric field profiles, comprising significantly higher energy levels (as much as 100 times greater) as compared to the IRE process, to directly and completely disintegrate the cell membrane rather than to electroporate the cell membrane. Such electric field profiles are not possible using currently available IRE equipment and protocols. The inability of current IRE methods and energy protocols to deliver the energy necessary to cause EMB explains why IRE treated specimens have never shown the pathologic characteristics of EMB treated specimens, and is a critical reason why EMB had not until now been recognized as an alternative method of cell destruction.

An embodiment of the system according to the present invention may comprise a software and hardware system for detecting and measuring a mass of target tissue in the prostate, breast, pancreas, skin, or other treatment area of a patient, for designing an EMB treatment protocol to ablate said mass, and for applying said EMB treatment protocol in an outpatient, doctor's office, or intra-operative hospital setting. An embodiment of the system of the invention includes an EMB pulse generator 16, one or more EMB treatment probes 20, one or more trackable biopsy needles 200 and one or more temperature probes 22. The embodiment of the system further employs a software-hardware controller unit (SHCU) operatively connected to said generator 16, probes 20, biopsy needles 200 and temperature probe(s) 22, along with one or more optional devices such as trackable anesthesia needles 300, endoscopic imaging scanners, ultrasound scanners, and/or other imaging devices or energy sources, and operating software for controlling the operation of each of these hardware devices.

### BRIEF DESCRIPTION OF THE DRAWINGS

FIG 1 is a diagram of a cell membrane pore.
FIG 2 is a diagram of cell membrane pore formation by a prior art method.
FIG. 3 is a schematic diagram of the software and hardware system according to the present disclosure.
FIG. 4A is a comparison of a prior art charge reversal with an instant charge reversal according to an embodiment of the present disclosure.
FIG. 4B is a square wave from instant charge reversal pulse according to an embodiment of the present disclosure.
FIG. 5 is a diagram of the forces imposed on a cell membrane as a function of electric field pulse width according to an embodiment of the present disclosure.
FIG. 6 is a diagram of a prior art failure to deliver prescribed pulses due to excess current.
FIG. 7A is a schematic diagram depicting a TRUSS scan of a suspect tissue mass.
FIG. 7B is a schematic diagram depicting the results of a 3D Fused Image of a suspect tissue mass.

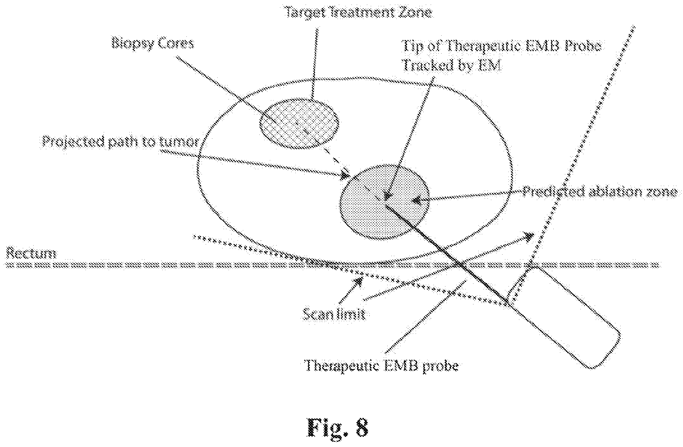

FIG. 8 is a schematic diagram depicting the target treatment area and Predicted Ablation Zone relative to a therapeutic EMB treatment probe 20 prior to delivering treatment.
FIG. 9 is a schematic diagram of a pulse generation and delivery system embodiment of the present r disclosure.
FIG. 10 is a diagram of the parameters of a partial pulse train according to an embodiment of the present disclosure.

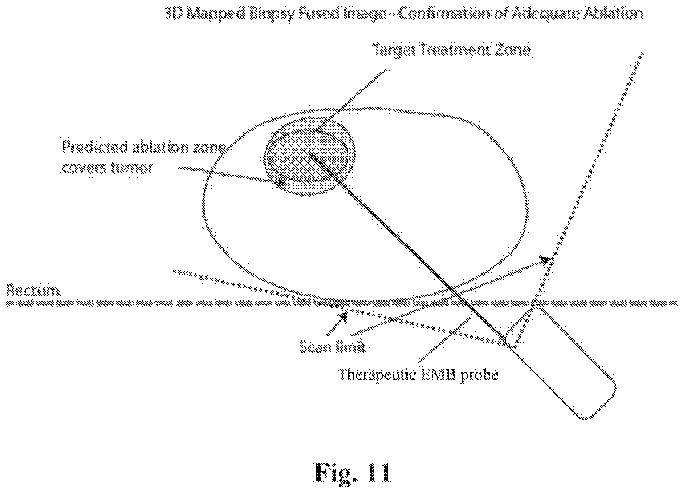

FIG. 11 is a schematic diagram depicting the target treatment area and Predicted Ablation Zone relative to a therapeutic EMB treatment probe 20 at the start of treatment delivery.
FIG. 12A is a schematic diagram of a therapeutic EMB treatment probe 20 according to one embodiment of the present disclosure.
FIG. 12B is a composite schematic diagram (1, 2 and 3) of the therapeutic EMB treatment probe 20 of FIG. 12A showing insulating sheath 23 in various stages of retraction.
FIG. 12C is a composite schematic diagram (1 and 2) of a therapeutic EMB treatment probe 20 according to another embodiment of the present disclosure.
FIG. 12D is a composite schematic diagram (1 and 2) of the therapeutic EMB treatment probe 20 of FIG. 12C showing insulating sheath 23 in various stages of retraction.
FIG. 13 is a schematic diagram of the enhanced trackable biopsy needle 200 according to an embodiment of the present disclosure.
FIG. 14 is a schematic diagram of the enhanced trackable anesthesia needle 300 according to an embodiment the present disclosure.
FIG. 15 is a schematic diagram depicting the positioning of a therapeutic EMB treatment probe 20 according to an embodiment of the present disclosure proximate the treatment area 2.
FIG. 16 is a schematic diagram depicting the positioning of a therapeutic EMB treatment probe 20 comprising a thermocouple 7 according to another embodiment of the present disclosure proximate the treatment area 2.
FIG. 17 is a schematic diagram depicting the positioning of a therapeutic EMB treatment probe 20 comprising a side port 8 for exposure of needle 9 according to another embodiment of the present disclosure proximate the treatment area 2.
FIG. 18 is a. schematic diagram depicting the positioning of a therapeutic EMB treatment probe 20 comprising a unipolar electrode 11 according to another embodiment of the present disclosure proximate the treatment area 2.
FIG. 19 is a schematic diagram depicting the positioning of a therapeutic EMB treatment probe 20 comprising a side port 8 for exposure of electrode-bearing needle 17 according to another embodiment of the present disclosure proximate the treatment area 2.
FIG. 20 is a schematic diagram depicting the positioning of a therapeutic EMB treatment probe 20 according to another embodiment of the present disclosure inside a cavity 400 in the human body.
FIG. 21 is a schematic diagram depicting the positioning of a therapeutic EMB treatment probe 20 comprising an expandable stabilizing balloon 27 according to another embodiment of the present disclosure inside a cavity 400 in the human body.
FIG. 22 is a schematic diagram depicting the positioning of a therapeutic EMB treatment probe 20 comprising an expandable electrode-bearing balloon 27 according to another embodiment of the present disclosure inside a cavity 400 in the human body.
FIG. 23 is a schematic diagram depicting the positioning of a therapeutic EMB treatment probe 20 according to another embodiment of the present disclosure inside a cavity 400 in the human body.
FIG. 24 is a schematic diagram depicting the use of two therapeutic EMB treatment probes 20 for deli very of EMB treatment.
FIG. 25 is a schematic diagram depicting the positioning of a therapeutic EMB treatment probe 20 delivered endoscopically using endoscopic ultrasound as guidance according to another embodiment of the present disclosure.
FIG. 26 is a schematic diagram depicting the positioning of a therapeutic EMB treatment probe 20 delivered endoscopically using endoscopic ultrasound as guidance according to another embodiment of the present disclosure.

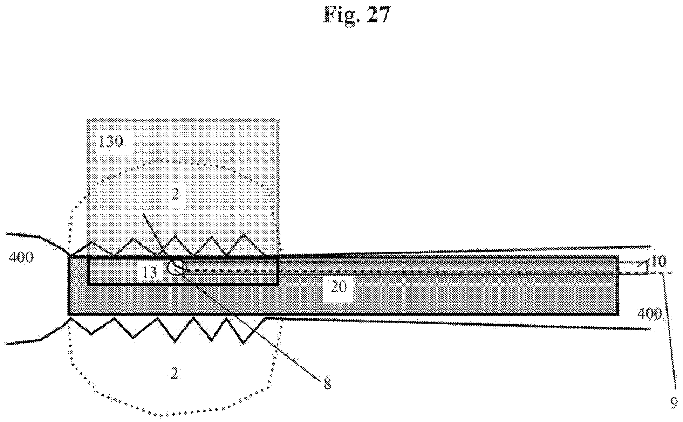

FIG. 27 is a schematic diagram depicting the positioning of a therapeutic EMB treatment catheter type probe 20 comprising an ultrasound transducer according to another embodiment of the present disclosure proximate the treatment area 2.

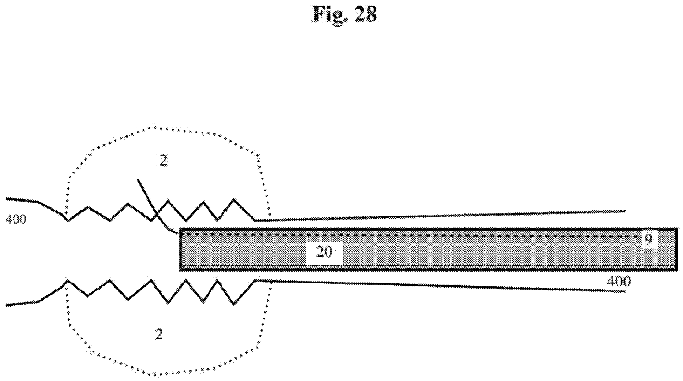

FIG. 28 is a schematic diagram depicting the positioning of a therapeutic EMB treatment catheter type probe 20 wherein needle 9 exits the distal end of catheter probe 20 according to another embodiment of the present disclosure proximate the treatment area 2.
FIG. 29 is a composite (A & B) schematic diagram depicting the positioning of a therapeutic EMB treatment probe 20 comprising an inflatable stent 19 according to another embodiment of the present disclosure inside a cavity 400 in the human body.
FIG. 30 is a schematic diagram depicting the positioning of a stent 19 left by EMB treatment probe 20 inside a cavity 400 in the human body.

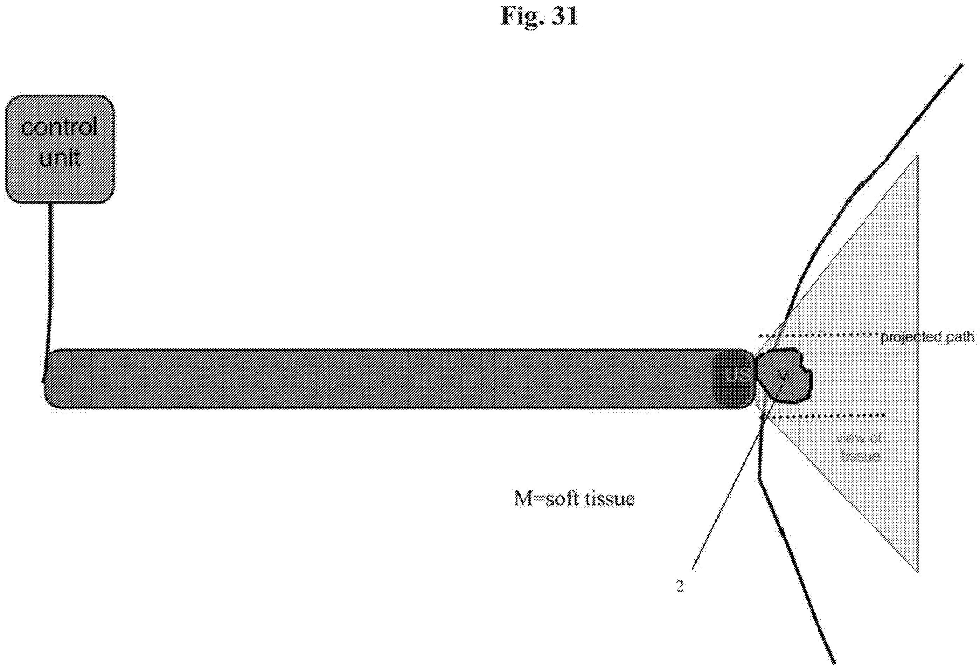

FIG. 31 is a schematic diagram depicting a US scan of a suspect tissue mass.

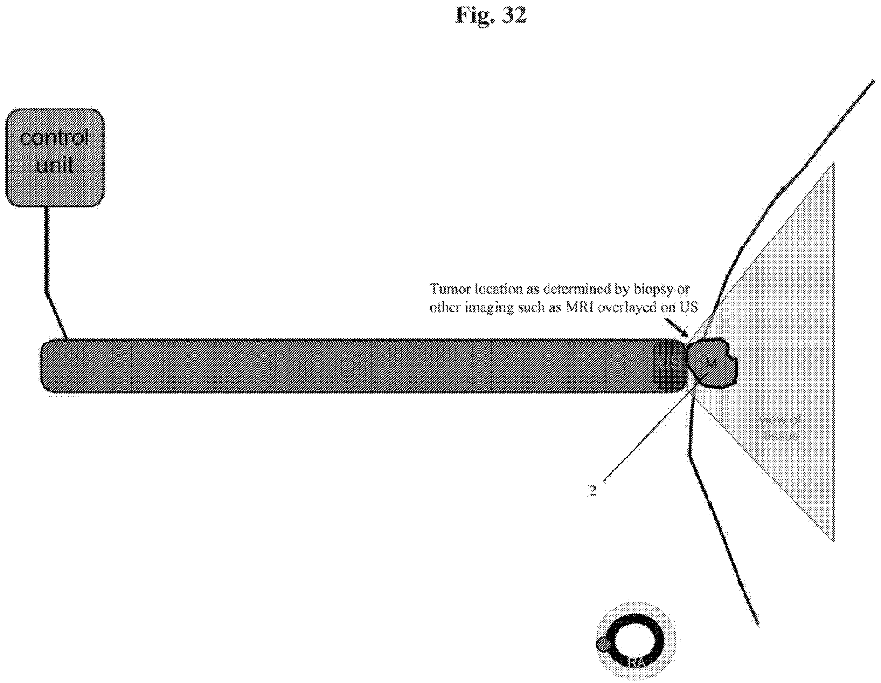

FIG. 32 is a schematic diagram depicting the results of a 3D Fused image of a suspect tissue mass.

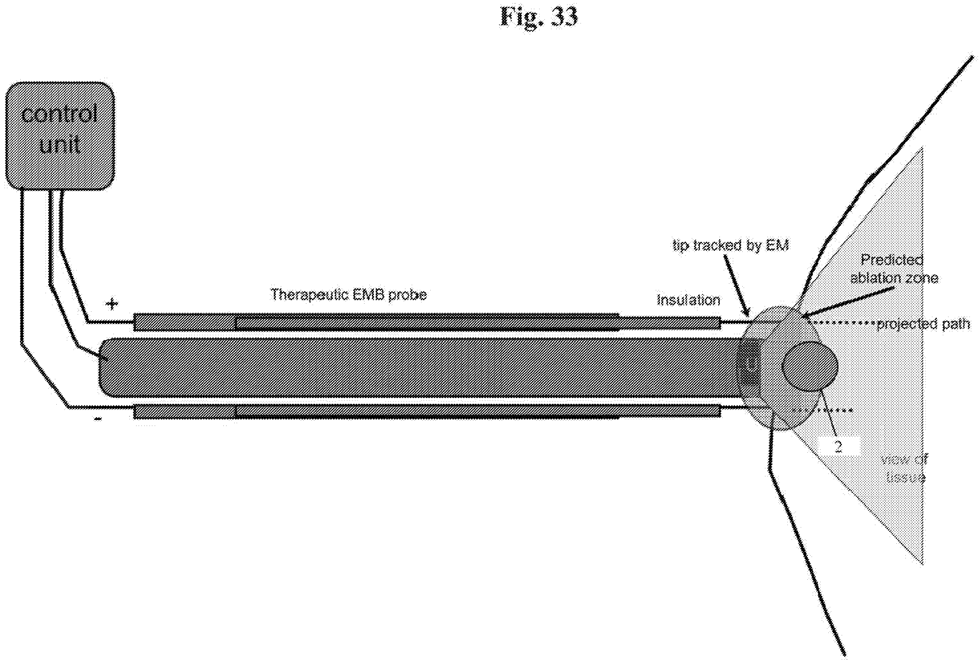

FIG. 33 is a schematic diagram depicting the target treatment area and Predicted Ablation Zone relative to a therapeutic EMB treatment probe 20 prior to delivering treatment.

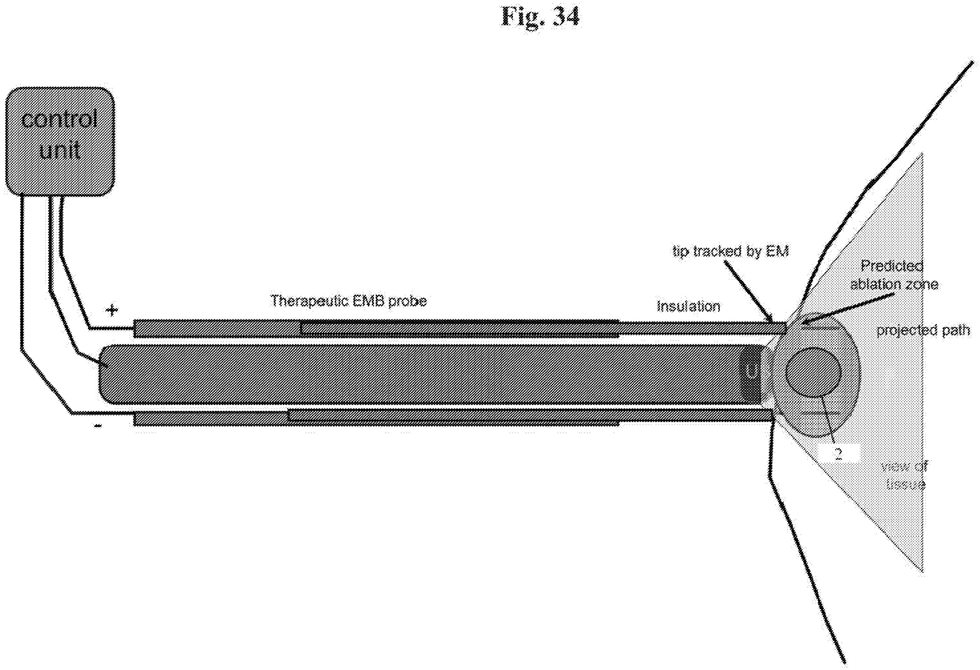

FIG. 34 is a schematic diagram depicting the target treatment area and Predicted Ablation Zone relative to a therapeutic EMB treatment probe 20 at the start of treatment delivery.

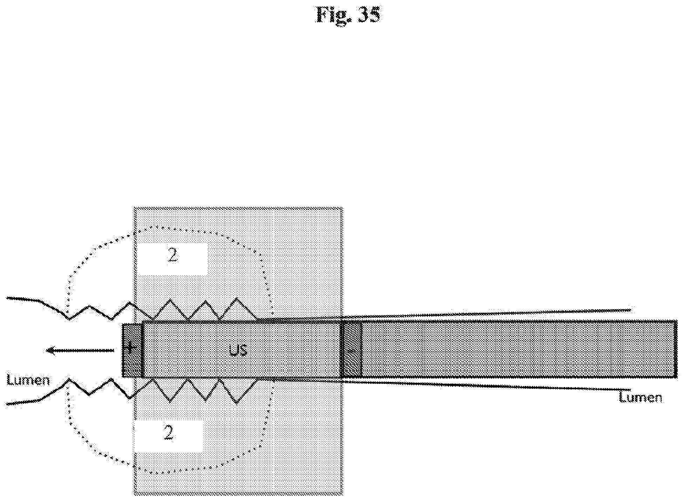

FIG. 35 is a schematic diagram depicting a US scan of a suspect tissue mass with the EMB catheter probe 20 with integrated US being moved into place.

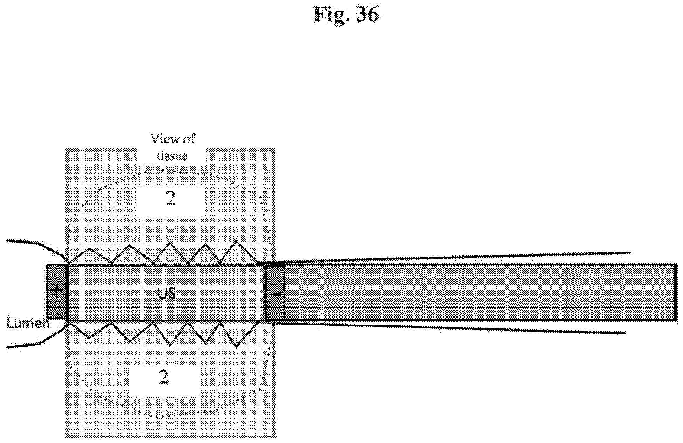

FIG. 36 is a schematic diagram depicting the results of a 3D fused Image of a suspect tissue mass.

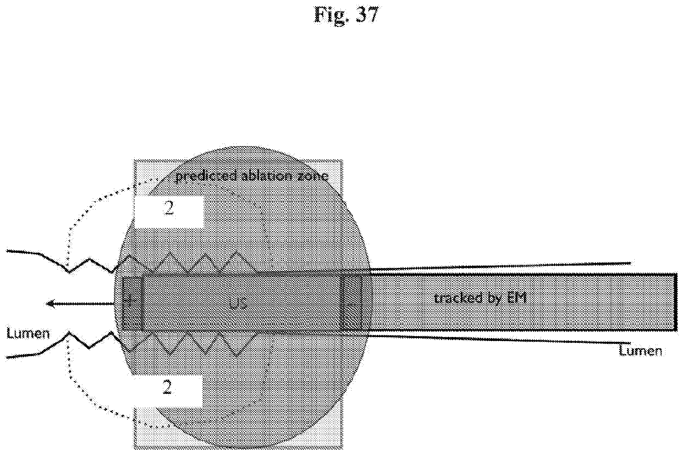

FIG. 37 is a schematic diagram depicting the target treatment area and Predicted Ablation Zone relative to a therapeutic EMB treatment probe 20 prior to delivering treatment.

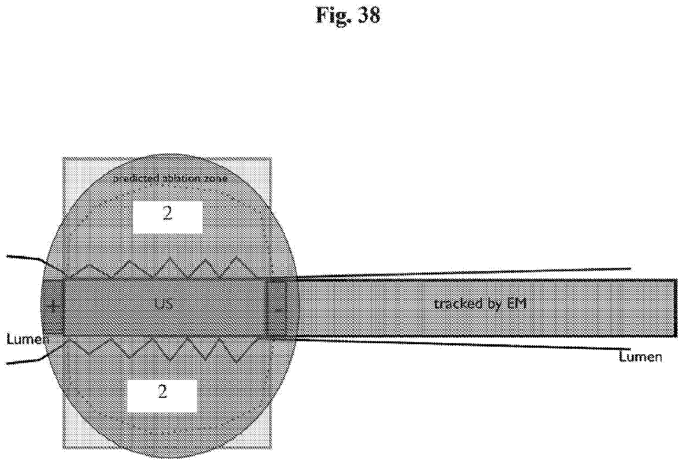

FIG. 38 is a schematic diagram depicting the target treatment area and Predicted Ablation Zone relative to a therapeutic EMB treatment probe 20 at the start of treatment delivery.
FIG. 39 is a schematic diagram depicting the positioning of a therapeutic EMB treatment catheter type probe 20 comprising an ultrasound transducer according to another embodiment of the present disclosure proximate the treatment area 2.
FIG. 40 is a schematic diagram depicting the. positioning of a therapeutic EMB treatment catheter type probe 20 wherein needle 9 exits the distal end of catheter probe 20 according to another embodiment of the present disclosure proximate the treatment area 2.

### DETAILED DESCRIPTION

In general, the software-hardware controller unit (SHCU) operating the proprietary treatment system software according to an embodiment of the present invention facilitates the treatment of cancerous tissue, sarcoma, soft tissue tumors, or neoplastic or unwanted tissue by directing the placement of EMB treatment probe(s) 20, biopsy needle(s) 200 and, optionally, anesthesia needle(s) 300, and by delivering electric pulses designed to cause EMB within the target tissue to EMB treatment probe(s) 20, all while the entire process may be monitored in real time via one or more two- or three-dimensional imaging devices and via. one or more biopsy samples taken at strategic locations to measure cell death. The system is such that the treatment may be performed by a physician under the guidance of the software, or may be performed completely automatically, from the process of imaging the treatment area to the process of placing one or more probes using robotic arms operatively connected to the SHCU to the process of delivering electric pulses and monitoring the results of same. Specific components of embodiments of the invention will now be described in greater detail.

### EMB Pulse Generator 16

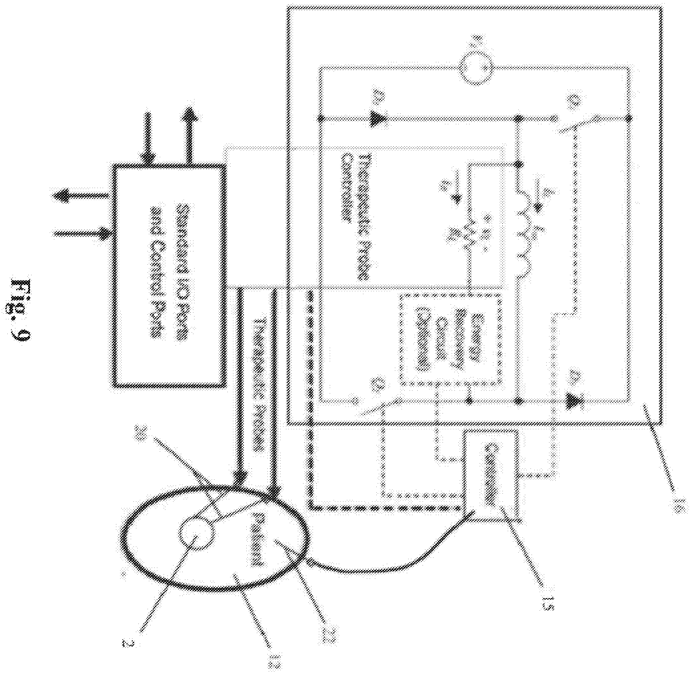

FIG. 9 is a schematic diagram of a system for generation of the electric field necessary to induce EMB of cells 2 within a patient 12. The system includes the EMB pulse generator 16 operatively coupled to Software Hardware Control Unit (SHCU) 14 for controlling generation and delivery to the EMB treatment probes 20 (two are shown) of the electrical pulses necessary to generate an appropriate electric field to achieve EMB. FIG. 9 also depicts optional onboard controller 15 which is preferably the point of interface between EMB pulse generator 16 and SHCU 14. Thus, onboard controller 15 may perform functions such as accepting triggering data from SHCU 14 for relay to pulse generator 16 and providing feedback to SHCU regarding the functioning of the pulse generator 16. The EMB treatment probes 20 (described in greater detail below) are placed in proximity to the soft tissue or cancerous cells 2 which are intended to be ablated through the process of EMB and the bipolar pulses are shaped, designed and applied to achieve that result in an optimal fashion. A temperature probe 22 may he provided for percutaneous temperature measurement and feedback to the controller of the temperature at, on or near the electrodes. The controller may preferably include an onboard digital processor and a memory and may be a general purpose computer system, programmable logic controller or similar digital logic control device. The controller is preferably configured to control the signal output, characteristics of the signal generation including the voltage, frequency, shape, polarity and duration of pulses as well as the total number of pulses delivered in a pulse train and the duration of the inter pulse burst interval.

With continued reference to FIG. 9, the EMB protocol calls for a series of short and intense bi-polar electric pulses delivered from the pulse generator through one or more EMB treatment probes 20 inserted directly into, or placed around the target tissue 2. The bi-polar pulses generate an oscillating electric field between the electrodes that induce a similarly rapid and oscillating buildup of transmembrane potential across the cell membrane. The built up charge applies an oscillating and flexing force to the cellular membrane which upon reaching a critical value causes rupture of the membrane and spillage of the cellular content. Bipolar poises are more lethal than monopolar pulses because the pulsed electric field causes movement of charged molecules in the cell membrane and re versal in the orientation or polarity of the electric field causes a corresponding change in the direction of movement of the charged molecules and of the forces acting on the cell. The added stresses that are placed on the cell membrane by alternating changes in the movement of charged molecules create additional internal and external changes that cause indentations, crevasses, rifts and irregular sudden tears in the cell membrane causing, more extensive, diverse and random damage, and disintegration of the cell membrane.

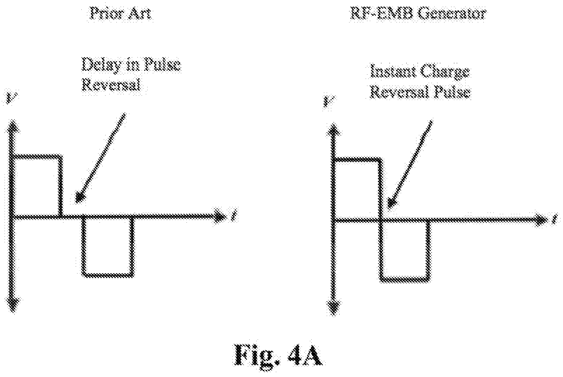

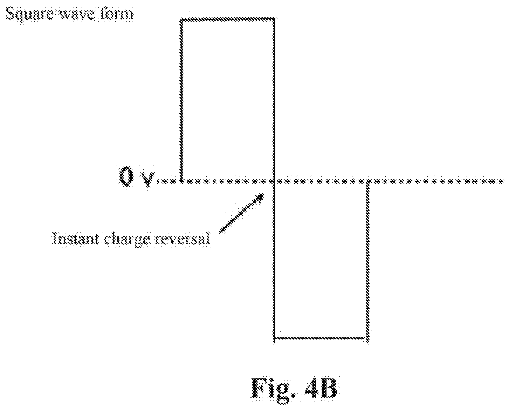

With reference to FIG. 4B, in addition to being bi-polar, the preferred embodiment of electric pulses is one for which the voltage over time traces a square wave form and is characterized by instant charge reversal pulses (ICR). A square voltage wave form is one that maintains a substantially constant voltage of not less than 80% of peak voltage for the duration of the single polarity portion of the trace, except during the polarity transition. An instant charge reversal pulse is a pulse that is specifically designed to ensure that substantially no relaxation time is permitted between the positive and negative polarities of the bi-polar pulse (See FIG. 4A). That is, the polarity transition happens virtually instantaneously.

The destruction of dielectric cell membranes through the process of Electrical Membrane Breakdown is significantly more effective if the applied voltage pulse can transition from a positive to a negative polarity without delay in between. Instant charge reversal prevents rearrangement of induced surface charges resulting in a short state of tension and transient mechanical forces in the cells, the effects of which are amplified by large and abrupt force reversals. Alternating stress on the target cell that causes structural fatigue is thought to reduce the critical electric field strength required, for EMB. The added structural fatigue inside and along the cell membrane results in or contributes to physical changes in the structure, of the cell. These physical changes and defects appear in response to the force applied with the oscillating EMB protocol and approach dielectric membrane breakdown as the membrane position shifts in response to the oscillation, up to the point of total membrane rupture and catastrophic discharge. This can be analogized to fatigue or weakening of a material caused by progressive and localized structural damage that occurs when a material is subjected to cyclic loading, such as for example a metal paper clip that is subjected to repeated bending. The nominal maximum stress values that cause such damage may be much less than the strength of the material under ordinary conditions. The effectiveness of this waveform compared to other pulse waveforms can save up to 1/5 or 1/6 of the total energy requirement.

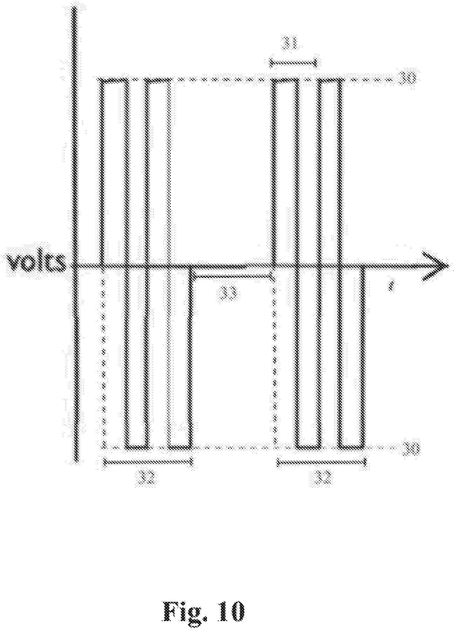

With reference to FIG. 10, another important characteristic of the applied electric field is the field strength (Volts/cm) which is a function of both the voltage 30 applied to the electrodes by the pulse generator 16 and the electrode spacing. Typical electrode spacing for a bi-polar, needle type probe might be 1 cm, while spacing between multiple needle probe electrodes can be selected by the surgeon and might typically be from 75 cm to 13 cm. A pulse generator for application of the present invention is capable of delivering up to a 10 kV potential. The actual applied field strength will vary over the course of a treatment to control circuit amperage which is the controlling factor in heat generation, and patient safety (preventing large unanticipated current flows as the tissue impedance falls during a treatment). Where voltage and thus field strength is limited by heating concerns, the duration of the treatment cycle may be extended to compensate for the diminished charge accumulation. Absent thermal considerations, a preferred field strength tor EMB is in the range of 1,500 V/cm to 10,000 V/cm.

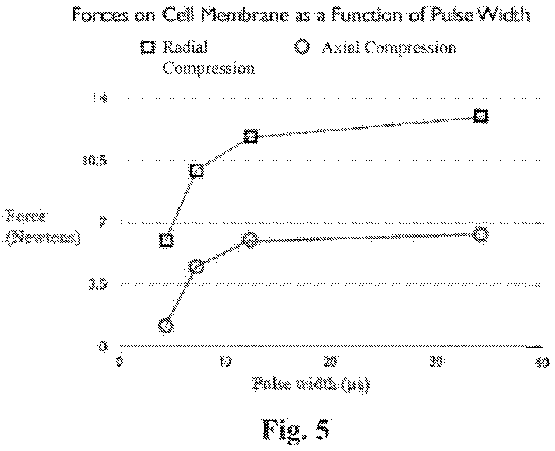

With continued reference to FIG. 10, the frequency 31 of the electric signal supplied to the EMB treatment probes 20, and thus of the field polarity oscillations of the resulting electric field, influences the total energy imparted on the subject tissue and thus the efficacy of the treatment but are less critical than other characteristics. A preferred signal frequency is from 14.2 kHz to less than 500 kHz. The lower frequency bound imparts the maximum energy per cycle below which no further incremental energy deposition is achieved. With reference to FIG. 5, the upper frequency limit is set based on the observation that above 500 KHz, the polarity oscillations are too short to develop enough motive force on the cell membrane to induce the desired cell membrane distortion and movement. More specifically, at 500 kHz the duration of a single full cycle is 2 µs of which half is of positive polarity and half negative. When the duration of a single polarity approaches 1 µs there is insufficient time for charge to accumulate and motive force to develop on the membrane. Consequently, membrane movement is reduced or eliminated and EMB does not occur. In a more preferred embodiment the signal frequency is from 100 kHz to 450 kHz. Here the tower hound is determined by a desire to avoid the need for anesthesia or neuromuscular-blocking drugs to limit or avoid the muscle contraction stimulating effects of electrical signals applied to the body. The upper bound in this more preferred embodiment is suggested by the frequency of radiofrequency thermal ablation equipment already approved by the FDA, which has been deemed safe for therapeutic use in medical patients.

In addition, the energy profiles that are used to create EMB also avoid potentially serious patient risks from interference with cardiac sinus rhythm, as well as localized barotrauma, which can occur with other therapies.

### EMB Treatment Probes 20

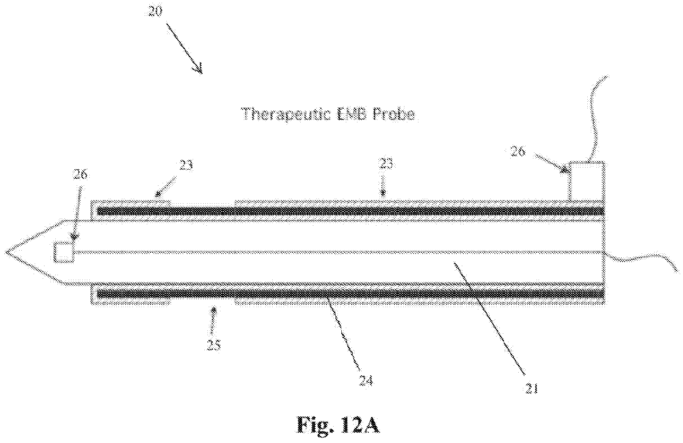

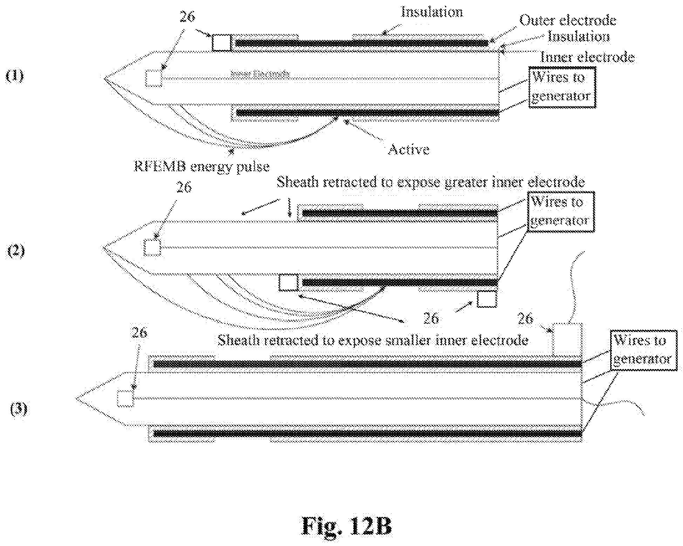

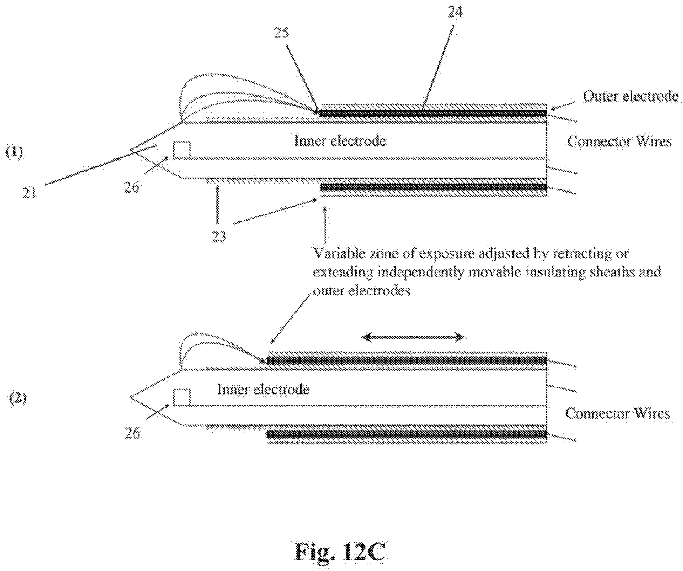

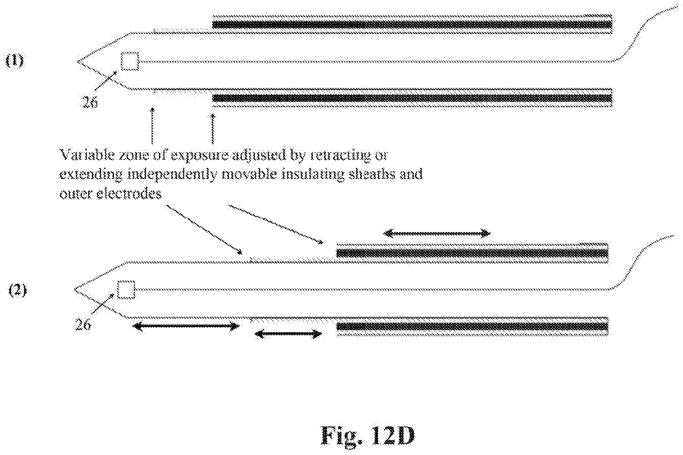

FIGs. 12A-12D depict a first set of embodiments of a therapeutic EMB treatment probe 20 envisioned specifically for use in the treatment of prostate cancer, pancreatic cancer, breast cancer, melanoma, sarcoma, soft tissue tumors or other skin .malignancies, although, it will be understood that the probe depicted in FIGs. 12A-12D may have additional applications outside these areas. With reference to FIGs. 12A-12B, the core (or inner electrode) 21 of EMB treatment probe 20 is preferably a needle of gage 17-22 with, a length of 5-25cm, and may be solid or hollow. Core 21 is preferably made of an electrically conductive material, such as stainless steel, and may additionally comprise one or more coatings of another conductive material, such as copper or gold, on the surface thereof. As shown in FIGs. 12A-12D, in the instant embodiment, the core 21 of treatment probe 20 has a pointed tip, wherein the pointed shape may be a 3-sided trocar point or a beveled point; however, in other embodiments, the tip may be rounded or flat. Treatment probe 20 further comprises an outer electrode 24 covering core 21 on at least one side. In a preferred embodiment, outer electrode 24 is also a cylindrical member completely surrounding the diameter of core 21. An insulating sheath 23, made of an inert material compatible with bodily tissue, such as Teflon^{®} or Mylar^{®}, is disposed around the exterior of core 21 and isolates core 21 from outer electrode 24. In this preferred embodiment, insulating sheath 23 is also a cylindrical body surrounding the entire diameter of core 21 and completely encapsulating outer electrode 24 except at active area 25, where outer electrode 24 is exposed directly to the treatment area. In an alternate embodiment, shown in FIGs. 12C-12D, insulating sheath 23 comprises two solid cylindrical sheaths wherein the outer sheath completely encapsulates the lateral area of outer electrode 24 and only the distal end of outer electrode 24 is exposed to the treatment area as active area 25. Insulating sheath 23 and outer electrode 24 are preferably movable as a unit along a lateral dimension of core 21 so that the surface area of core 21 that is exposed to the treatment area is adjustable, thus changing the size of the lesion created by the EMB pulses. FIGs. 12B(3) and 12C(2) depict insulating sheath 23 and outer electrode 24 advanced towards the pointed tip of core 21, defining a relatively small treatment area, while FIGs. 12B(2) and 12C(1) depict insulating sheath 23 and outer electrode 24 retracted to define a relatively large treatment area. Electromagnetic (EM) sensors 26 on both core 21 and insulating sheath 23/outer electrode 24 member send information to the Software Hardware Controller Unit (SHCU) for determining the relative positions of these two elements and thus the size of the treatment area, preferably in real time. EM sensors 26 may be a passive EM tracking sensor/field generator, such as the. EM tracking sensor manufactured by Traxtal Inc. Alternatively, instead of utilizing EM sensors, EMB treatment probes 20 may be tracked in real time and guided using endoscopy, ultrasound or other imaging means known in the art.

One means for enabling the relative movement between core 21 and insulating sheath 23/outer electrode 24 member is to attach insulating sheath 23/outer electrode 24 member to a fixed member (i.e., a handle) at a distal end of probe 20 opposite the tip of core 21 by a screw mechanism, the turning of which would advance and retract the insulating sheath 23/outer electrode 24 member along the body of the core 21. Other means for achieving this functionality of EMB treatment probe 20 are known in the art.

One of conductive elements 21, 24 comprises a positive electrode, while the other comprises a negative electrode. Both core 21 and outer electrode 24 are connected to the EMB pulse generator 20 through insulated conductive wires, and which are capable of delivering therapeutic EMB pulsed radio frequency energy or biphasic pulsed electrical energy under sufficient conditions and with sufficient treatment parameters to achieve the destruction and disintegration of the membranes of cancer cells, or unwanted tissue, through the process of EMB, as described in more detail above. The insulated connection wires may either be contained within the interior of EMB treatment probes 20 or on the surface thereof However, EMB treatment probes 20 may also be designed to deliver thermal radio frequency energy treatment, if desired, as a complement to or instead of EMB treatment.

Alternatively, or in addition to the sensors described above, EMB treatment probes 20 may contain a thermocouple, such as a Type K- 40AWG thermocouple with Polyimide Primary/Nylon Bond Coat insulation and a temperature range of -40 to +180C, manufactured by Measurement Specialties. The lumen of the optional thermocouple may be located on EMB treatment probe 20 such that the temperature at the tip of the probe can be monitored and the energy delivery to probe 20 modified to maintain a desired temperature at the tip of probe 20.

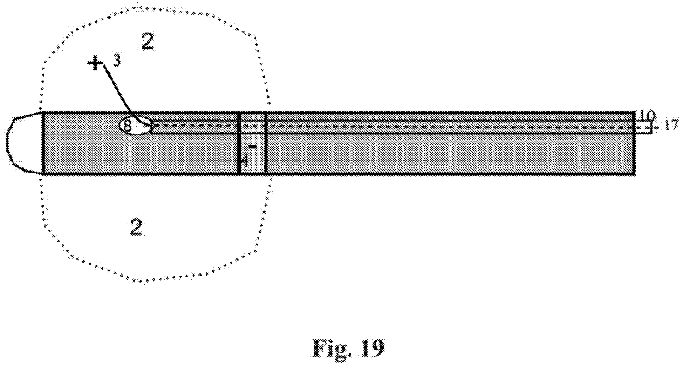

In an alternative embodiment of EMB treatment probes 20, one of either the positive (+) 3 or negative (-) 4 electrodes is on an outer surface of EMB treatment probe 20, while the other polarity of electrode is placed on the tip of a curved needle 17 inserted through a lumen 10 in the interior of core 21. Except for active surface 25 and a side hole 8, through which needle 17 may exit lumen 10, insulating sheath 23 may completely envelope probe 20 to isolate the two electrodes (see FIG. 19).

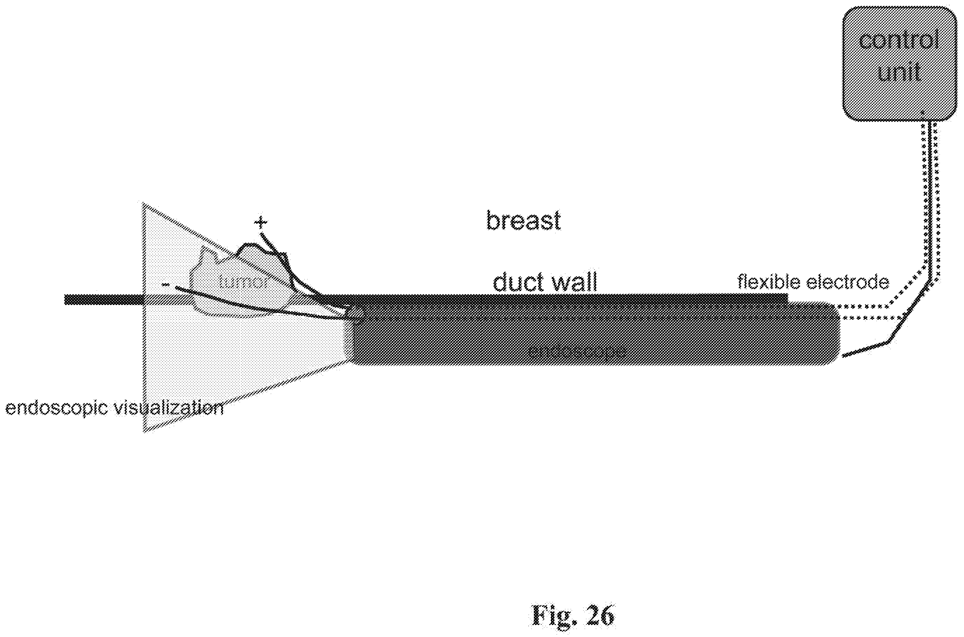

In yet another embodiment, the two curved needle electrodes can be placed through a scope and visualized as they extend out of the scope. For example, in the treatment of breast cancer, the two curved needle electrodes may, under direct scope visualization, pierce the walls of the breast duet and extend into the breast tissue (See FIG. 26).

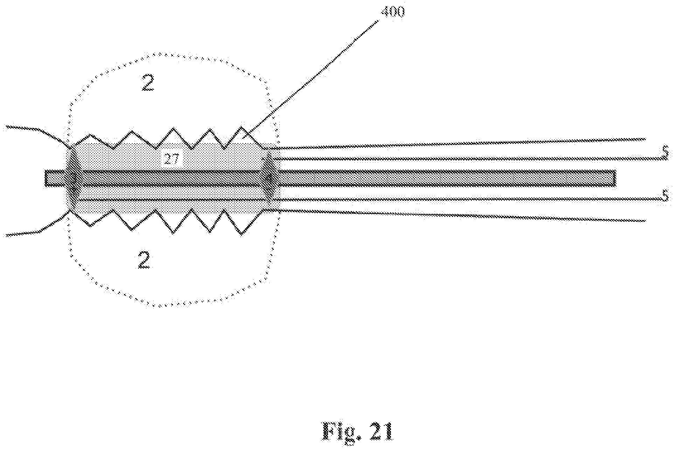

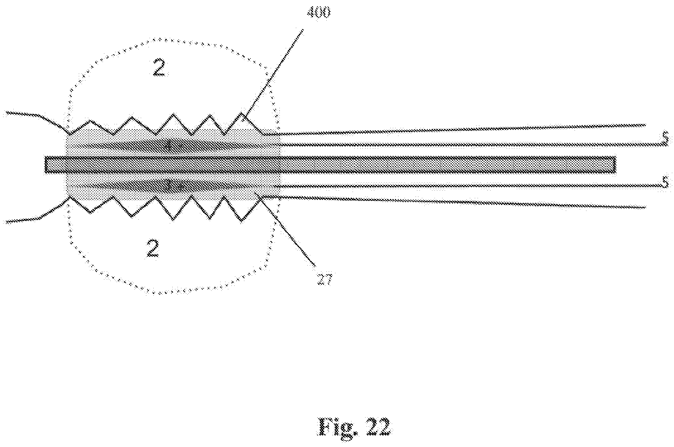

In yet another alternative embodiment of EMB treatment probes 20, unipolar or bipolar electrodes are placed on an expandable balloon 27, the inflation of which may he controlled by the SHCU via a pneumatic motor or air pump, etc, in this embodiment, when the balloon 27 is placed inside a cavity 400 in the human body (proximate a designated treatment area) and inflated, the electrodes on the balloon' s surface are forced against the wall of the cavity 400 to provide a path for current to flow between the positive and negative electrodes (see FIG.21). The positive and negative electrodes can have different configurations on the balloon 27, i.e., they may be arranged horizontally around the circumference of the balloon 27 as in FIG. 21, or longitudinally along the long axis of the balloon as in FIG. 22. In some embodiments, more than one each of positive and negative electrodes may be arranged on a single balloon.

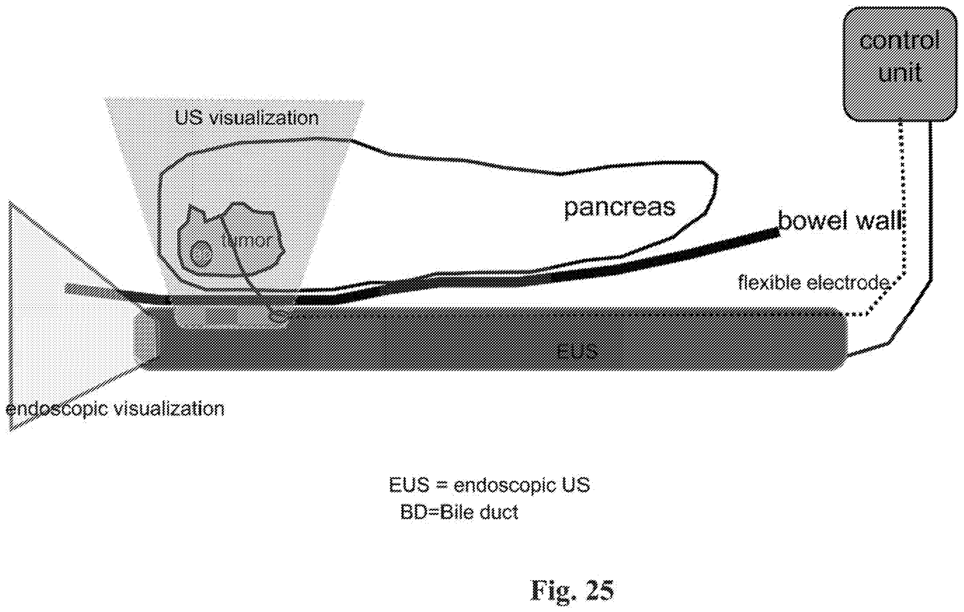

In another embodiment, depending on the location of the target tissue being treated, the EMB treatment probers) 20 can be configured to be delivered endoscopically using endoscopic ultrasound (US) as a guidance method. For example., in the treatment of pancreatic cancer as described herein, the probes may be placed through, the posterior stomach or duodenal wall with treatment administered to the pancreatic tissue involved by cancer (See FIG. 25).

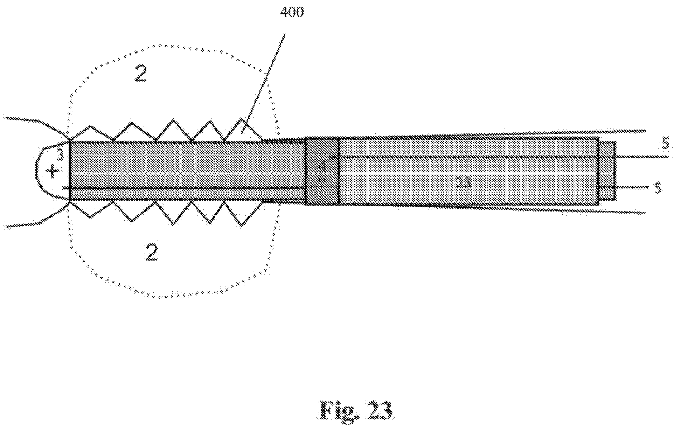

In another embodiment one electrode is on the end of a sheath through which the EMB treatment probe 20 is placed. By moving the catheter various distances from the end of the sheath, various distances between the electrodes can be accomplished thus changing the size and shape of the treatment zone (see FIG. 23).

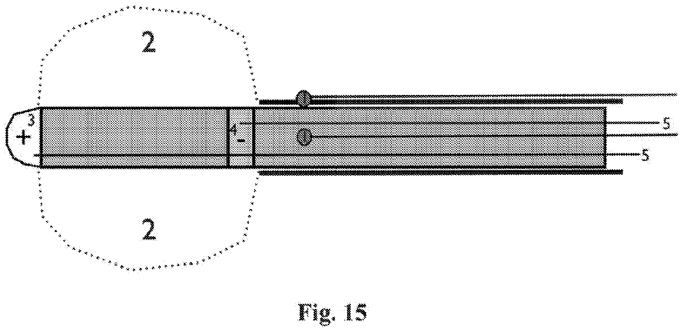

Another embodiment of treatment probe 20 is described with collective reference to FIGs. 15-17, 19-23, 29-30 and 39-40. As shown therein, EMB treatment probes may alternatively be comprised of at least one therapeutic catheter-type probe 20 capable of delivering therapeutic EMB pulsed radio frequency energy or biphasic pulsed electrical energy under sufficient conditions and with sufficient treatment parameters to completely break down the membranes of carcinoma, neoplastic cells or other unwanted tissue. Probes 20 are preferably of the catheter type known in the art and having one or more central lumens to, among other things, allow probe 20 to be placed over a guide wire for ease of insertion and/or placement of probe 20 within a cavity 400 of the human body according to the Seldinger technique. A catheter for this purpose may be a Foley-type catheter, sized between 10 French to 20 French and made of silicone, latex or any other biocompatible, flexible material.

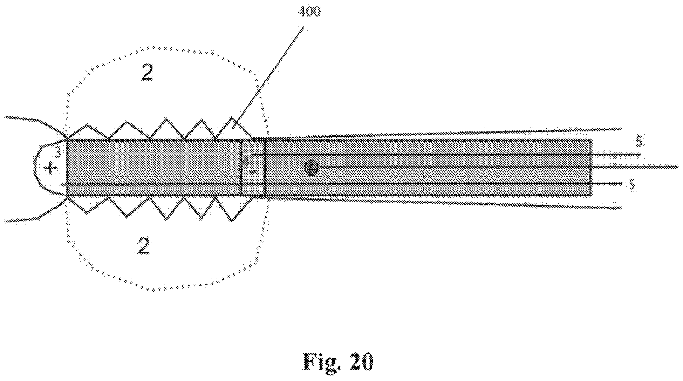

In a preferred embodiment, illustrated in FIG. 20, probe 20 former comprises one positive 3 and one negative 4 electrode disposed on an outer surface of probe 20 and spaced apart by a distance along the longitudinal axis of probe 20 such that current sufficient to deliver the EMB pulses described herein may be generated between the electrodes 3, 4. The spacing between positive 3 and negative 4 electrodes may vary by design preference, wherein a larger distance between electrodes 3, 4 provides a larger treatment area 2. FIG. 20 depicts electrodes 3, 4 on an outer surface of probe 20; alternatively, electrodes 3, 4 are integral to the surface of probe 20. In yet another embodiment, as shown in FIG. 23, one of electrodes 3, 4 (negative electrode 4 as shown in FIG. 23) may be placed on the end of an insulated sheath 23 that either partially or fully surrounds probe 20 along a radial axis thereof and is movable along a longitudinal axis of probe 20 relative to the tip thereof (on which positive electrode 3 is located as shown in FIG. 23) to provide even further customizability with respect to the distance between electrodes 3, 4 and thus the size of treatment area 2. As above, insulating sheath 23 is preferably made of an inert material compatible with bodily tissue, such as Teflon^{®} or Mylar^{®}, Means for enabling the relative movement between probe 20 and insulating sheath 23 include attaching insulating sheath 23 to a fixed member (i.e., a handle) at a distal end of probe 20 opposite the tip of probe 20 by a screw mechanism, the taming of which would advance and retract the insulating sheath 23 along the body of the probe 20, Other means for achieving this functionality of EMB treatment probe 20 are known in the art.

Without limitation, electrodes may be flat (i.e., formed on only a single side of probe 20), cylindrical and surrounding probe 20 around an axis thereof, etc. Electrodes 3, 4 are made of an electrically conductive material. Electrodes 3, 4 may be operatively connected to EMB pulse generator 16 via one or more insulated wires 5 for the delivery of EMB pulses from generator 16 to the treatment area 2. Connection wires 5 may either be intraluminal to the catheter probe 20 or extra-luminal on the surface of catheter probe 20.

Also in a preferred embodiment, as show in FIG. 20, probe 20 further comprises an electromagnetic (EM) sensor/transmitter 6 of the type described above. EM sensors 26 may be located on both probe 20 and optional insulating sheath 23 to send information to the Software Hardware Controller Unit (SHCU) for determining the positions and/or relative positions of these two elements and thus the size of the treatment area, preferably in real time. Alternatively, instead of utilizing EM sensors, EMB treatment probes 20 may be tracked in real time and guided using endoscopy, ultrasound or other imaging means known in the art.

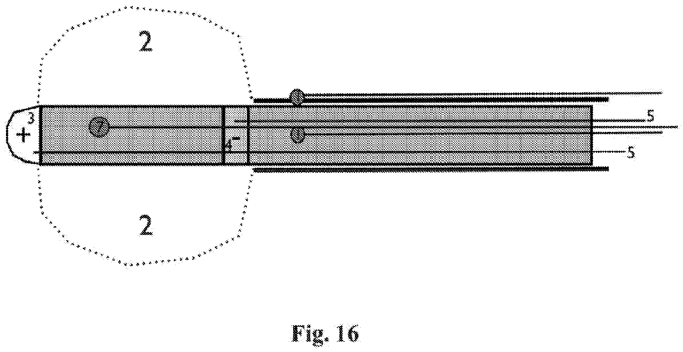

In a preferred embodiment, as shown in FIG. 16, probe 20 further comprises a thermocouple 7 of the type described above on the insulating surface thereof such that the temperature at the wall of the catheter can be monitored and the energy delivery to electrodes 3, 4 modified to maintain a desired temperature at the wall of the probe 20 as described in further detail above.

In yet another alternative embodiment of EMB treatment probes 20, unipolar or bipolar electrodes are placed on an expandable balloon 27, the inflation of which may be controlled by the SHCU via a pneumatic motor or air pump, etc. In this embodiment, when the balloon 27 is placed inside a cavity 400 in the human body (proximate a designated treatment area) and inflated, the electrodes on the balloon's surface are forced against the wall of the cavity 400 to provide a path for current to flow between the positive and negative electrodes (see FIG. 21). The positive and negative electrodes can have different configurations on the balloon 27, i.e., they may be arranged horizontally around the circumference of the balloon 27 as in FIG. 21, or longitudinally along the long axis of the balloon as in FIG. 22. In some embodiments, more than one each of positive and negative electrodes may be arranged on a single balloon.

In certain embodiments of the present invention, the EMB treatment probe 20 is inserted into the treatment area through a body cavity 400, such as the urethra for treatment of a cancerous mass 2 proximate the peri-urethral prostatic tissue. Optionally, the catheter may comprise a non-electrode-containing balloon that is otherwise of the general type described above on its distal end, such that when the balloon (not shown) is inflated, the catheter and EMB treatment probe 20 are anchored within the treatment area for the target tissue by a friction fit of the balloon within the body cavity 400.

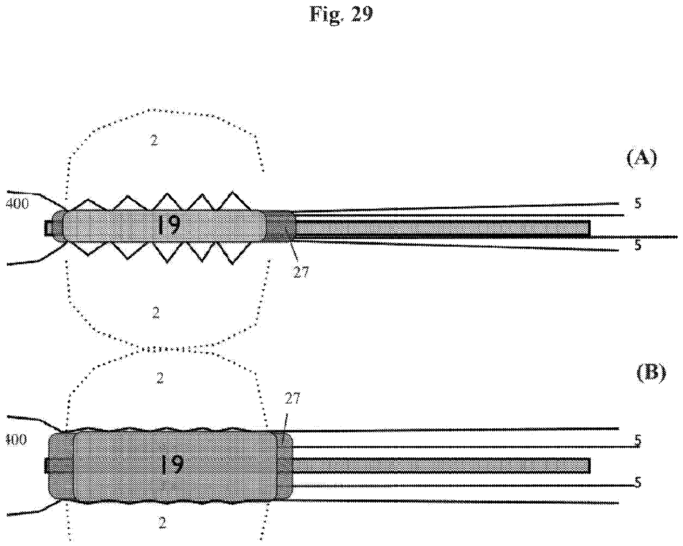

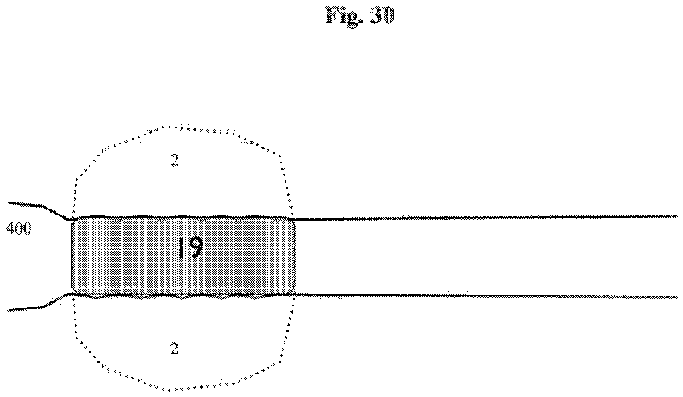

In yet another embodiment, EMB catheter-type probe 20 could deliver a stent 19 to the abnormal region / treatment area 2 in, i.e., the bile or pancreatic duct, which is associated with a narrowing causing obstruction. This configuration would allow the delivery of an EMB treatment protocol at the same time as stent 19 is used to expand a stricture in a lumen. Stent 19 may also comprise conducting and nonconducting areas which correspond to the unipolar or bipolar electrodes on EMB probe 20. An example treatment protocol would include placement of EMB probe 20 having balloon 27 with a stent 19 over the balloon 27 in its non-expanded state (FIG. 29(A)), expansion of balloon 27 which in turn expands stent 19 (FIG. 29(B)), delivery of the RFEMB treatment, and removal of the EMB treatment probe 20 and balloon 27, leaving stent 19 in place in the patient (see FIG. 30).

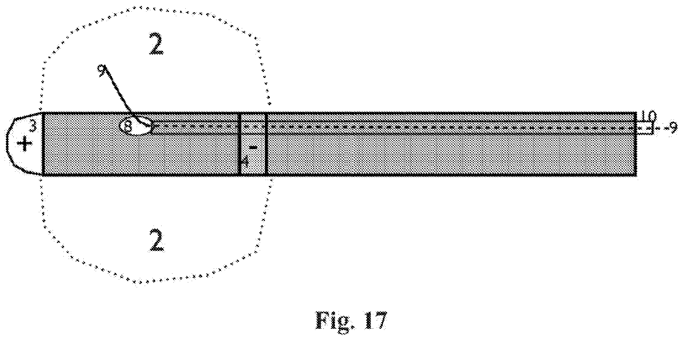

In an alternative embodiment of EMB treatment probes 20, one of either the positive (+) 3 or negative (-) 4 electrodes is on an outer surface of EMB treatment probe 20, while the other polarity of electrode is placed on the tip of a curved needle 9 inserted through an interior lumen 10 such as that described above (see FIG. 17).

In other embodiments, EMB treatment probe 20 is made contiguous with and/or held within a catheter, such as a Foley-type catheter as described above, for ease of insertion of EMB probe 20 into the treatment area. Alternatively, a catheter through which EMB probes 20 are inserted may serve as one of a pair of bipolar electrodes, while the EMB treatment probe 20 is placed directly within the target tissue to serve as the other electrode.

In some embodiments of the present invention, EMB treatment probes 20 contain sensors of the type described by Laufer et al. in "Tissue Characterization Using Electrical Impedance Spectroscopy Data: A Linear Algebra Approach", Physiol Meas. 33 (2012) 997-1013, to investigate tissue characteristics to determine cancerous from non-cancerous tissue. Alternatively, or in addition to sensors of the type described by Laufer, EMB treatment catheter type probes 20 may contain sensors to determine cellular content spillage as necessary to quantify cell death In the treatment area via EMB; one example of such a sensor is described by Miller et al. in "Integrated Carbon Fiber Electrodes Within Hollow Polymer Microneedles For Transdermal Electrochemical Sensing", Biomicrofluidics, 2011 Mar 30;5(1):13415. The sensors described herein may be placed anywhere on the EMB treatment probes, or inside a lumen therein.

Electrical membrane breakdown, unlike IRE or other thermal ablation techniques, causes immediate spillage of all intracellular components of the ruptured cells into an extracellular space and exposes the internal constituent parts of the cell membrane to the extracellular space. The intracellular components include cellular antigens and the internal constituent parts of the cell membrane include antigens specific to the cell membrane which induce an immunologic response to destroy and remove this and like material in the body of the subject. Like material may be other material in the body of the subject having the same cellular antigens or cell membrane specific antigens at locations remote from the treatment site including metastatic tissue. The immunologic response can be enhanced by administration of one or more drugs, materials or agents that increase the immunologic response process including drugs which block inhibition of the CTLA-4 inhibitory signal of cytotoxic lymphocytes or that binds to S100A9 and modulating regulatory myeloid cell functions.

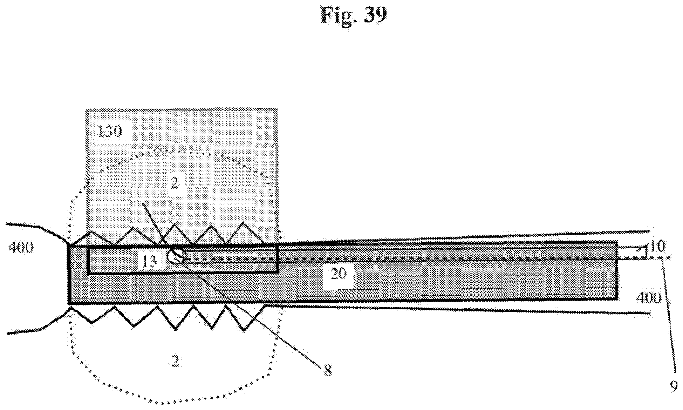

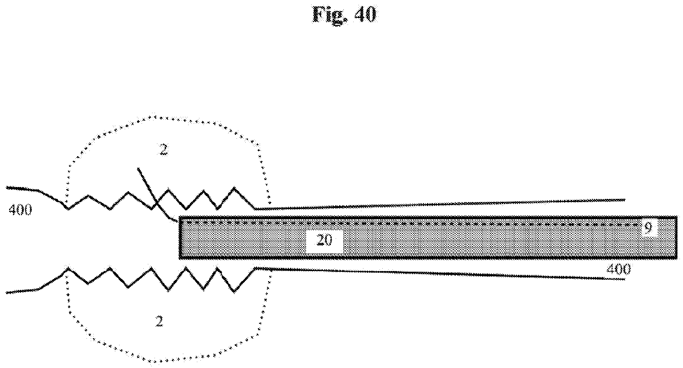

Thus, alternatively or in addition to the sensors described above, EMB treatment probes 20 preferably have a hollow interior defined by an inner lumen 10 (or, in the case of a catheter-type probe, an additional interior lumen 10) of sufficient diameter to accommodate a spinal needle 9 of one or more standard gauges to be inserted there through for the injection of adjuvant immunotherapy type drugs into the lesion formed by EMB treatment to enhance the immunologic response of said treatment (see FIG. 17). Alternatively, the inner lumen 10 may be sized to allow for the injection of biochemical or biophysical nano-materials there through into the EMB lesion to enhance the efficacy of the local ablative effect, or the immunologic response and effect of the EMB treatment, or to allow inj ection of reparative growth stimulating drugs, chemicals or materials. In a preferred embodiment, as shown in FIG. 17, interior lumen 10 terminates proximate an opening 8 in the side of probe 20 to allow needle 9 to exit probe 20 to access treatment area 2 for delivery of the drugs. In an alternative embodiment, shown in FIG. 40 in the case of a catheter-type probe 20 interior lumen 10 may terminate, and needle 9 may exit, with an opening at distal end of probe 20. In either case, probe 20 may further comprise an ultrasound transducer 13 at a distal end thereof (see FIG. 39) for the formation of an endoscopic viewing area 130 overlapping treatment area 2 to aid in guiding the needle 9 to the appropriate point in the patient for delivery of the drugs. Alternatively, needle 9 may be manipulated via visual guidance. To achieve these functions, needle 9 must be flexible and/or curved to allow it to locate and exit through opening 8 or the distal end of probe 20. Needle 9 is also preferably curved to allow it to pierce the wall of the surrounding tissue, such as bowel, duct or urethra, within the patient's body.

In another embodiment, interior lumen 10 may be sized to allow for the injection of biochemical or biophysical nano-materials there through into the EMB lesion to enhance the efficacy of the local ablative effect, or the immunologic response and effect of the EMB treatment, or to allow injection of reparative growth stimulating drugs, chemicals or materials.

A lumen 10 of the type described herein may also advantageously allow the collection and removal of tissue or intra-cellular components from the treatment area or nearby vicinity. This functionality may take the place of the trackable biopsy needle 200 described in more detail below, and can be used for such purposes before, during or after the application of EMB pulses from the EMB treatment probe 20.

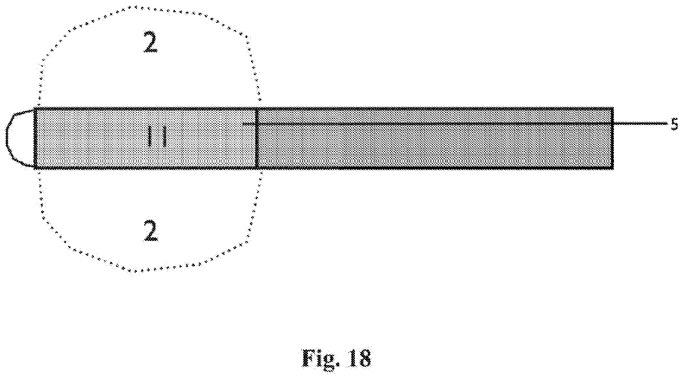

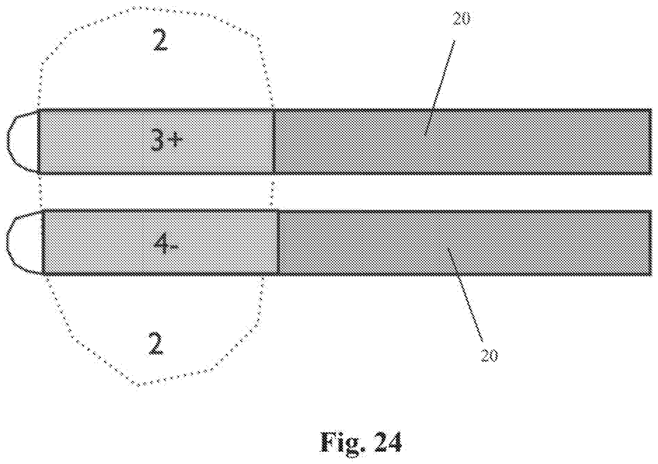

One of ordinary skill in the art will understand that the EMB treatment probe(s) 20 may take various forms provided that they are still capable of delivering EMB pulses from the EMB pulse generator 14 of the type, duration, etc. described above. For example, the EMB treatment probes 20 have been described herein as a rigid assembly, but may also be semi-rigid assembly with formable, pliable and/or deformable components. As another example, EMB treatment probes 20 may be unipolar 11 (see FIG. 18) and used with an indifferent electrode placed on a remote location from the area of treatment (see FIG. 18). In yet another embodiment, two EMB treatment probes 20 may be used, wherein each probe has one each of a positive and negative electrode (See FIG. 24).

It will also be understood that, instead of a EMB treatment probe having a lumen capable of providing a delivery path for immunologic response enhancing drugs, such drugs may be administered by any means, including without limitation, intravenously, orally or intramuscularly and may further be injected directly into or adjacent to the target soft tissue immediately before or after applying the EMB electric field. Such immunologic response enhancing drug may be comprised also of autologous dendritic cells.

### Trackable Biopsy Needles 200

Unlike irreversible electroporation, electrical membrane breakdown EMB causes immediate visually observable tissue changes which show cellular membrane destruction and immediate cell death. The present disclosure therefore includes the optional biopsy of a portion of the treated target tissue to verify treatment efficacy immediately upon completion of each tissue treatment during the ongoing therapy procedure, while the patient is still in position for additional, continued or further treatment.

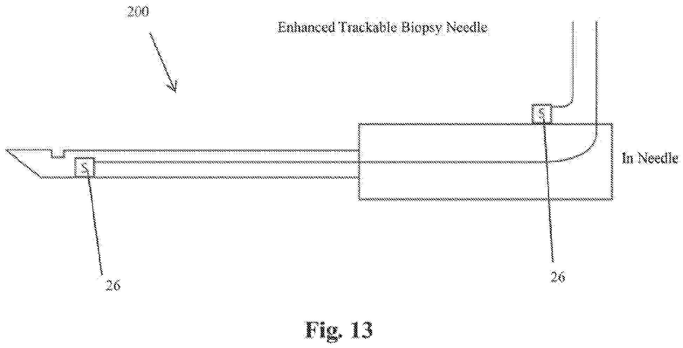

A biopsy needle 200 suitable for this purpose is shown in FIG. 13. Like EMB treatment probes 20, biopsy needle 200 may comprise sensor/transmitters 26 (electromagnetic or otherwise) built into the needle and/or needle body to track the location of die biopsy tip of needle 200 and/or the orientation of the needle 200 as a whole, in certain embodiments, biopsy needle 200 may also comprise sensors to investigate tissue characteristics to determine cancerous from non-cancerous tissue and/or determine cellular content spillage in order to ascertain and/or document cancer cell death, such as those sensors described by Laufer and Miller, above.

Biopsy needle 200 is preferably operatively connected to SBCU14 to provide real-time data from any sensors contained thereon and to enable real-time tracking of biopsy needle 200 by SBCU 14 to monitor treatment, as described in more detail below. Additional treatment may be immediately administered via, i.e., EMB treatment probe 20, based on the biopsy tissue inspection or result and/or other information obtained from the sensors on biopsy needle 200 or visual determination of treatment efficacy without removing biopsy needle 200 from the treatment area.

### Trackable Anesthesia Needles 300

EMB, by virtue of its bipolar wave forms in the described frequency range, does not cause muscle twitching and contraction. Therefore, a procedure using the same may be carried out under local anesthesia without the need for general anaesthesia and neuromuscular blockade to attempt to induce paralysis during the procedure. Rather, anesthesia can be applied locally for the control of pain without the need for the deeper and riskier levels of sedation.

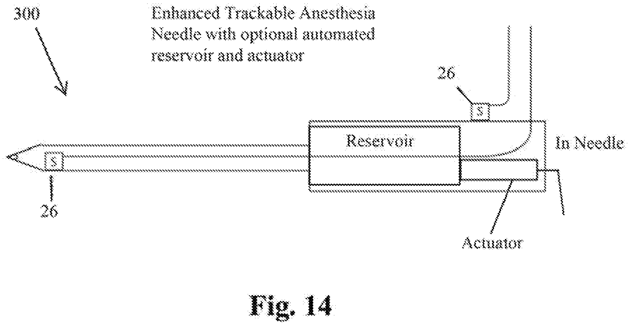

For this purpose, one or more trackable anesthesia needles 300 may he provided. With reference to FIG. 14, Anesthesia needles 300 may be of the type known in the art and capable of delivering anesthesia to the Neurovascular bundles or other potential treatment regions, including the point of entry of needle 300, EMB probe 20, biopsy probe 200 or any of the other devices described herein through the skin to enhance pain relief. Anesthesia needles 300 may also comprise sensor/transmitters 26 (electromagnetic or otherwise) built into the needle and/or needle body to track the location anesthesia needle 300. Anesthesia needles 300 are preferably operatively connected to SHCU 14 to enable real-time tracking of anesthesia needle 300 by SHCU 14 and/or to monitor administration of anesthesia, as described in more detail below.

Alternatively, trackable anesthesia needles 300 may be omitted in favor of conventional anesthesia needles which may be applied by the physician using conventional manual targeting techniques and using the insertion point insertion path and trajectories generated by the software as described in further detail below.

### Software Hardware Control Unit (SHCU) 14 and Treatment System Software

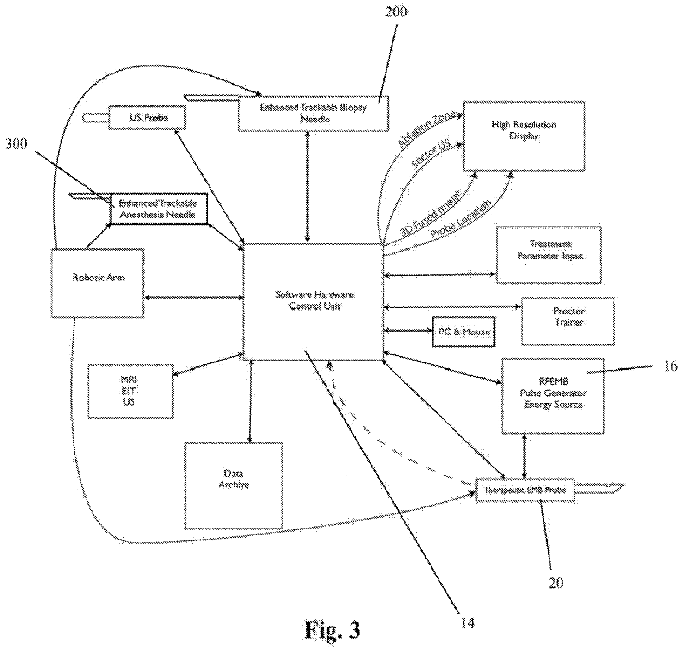

With reference to FIG. 3, the Software Hardware Control Unit (SHCU) 14 is operatively connected to one or more (and preferably all) of the therapeutic and/or diagnostic probes/needles, imaging devices and energy sources described herein namely, in a preferred embodiment, the SHCU 14 is operatively connected to one or more EMB pulse generator(s) 16, EMB treatment probe(s) 20, trackable biopsy needle(s) 200 and trackable anesthesia needle(s) 300 via electrical/manual connections for providing power to the connected devices as necessary and via data connections, wired or wireless, for receiving data transmitted by the various sensors attached to each connected device. SHCO 14 is preferably operatively connected to each of the devices described herein such as to enable SHCU 14 to receive all available data regarding the operation and placement of each of these devices. For example, SHCU 14 may be connected to one or more trackable anesthesia needles 300 via a fluid pump through which liquid medication, is provided to anesthesia needle 300 such that SHCU 14 may monitor and/or control the volume, rate, type, etc. of medication provided through needle(s) 300.

In an alternative embodiment, SHCU 14 is also connected to one or more of the devices herein via at least one robot arm such that SHCU 14 may itself direct the placement of various aspects of the device relative to a patient, potentially enabling fully automatized and robotic treatment of certain cancerous or unwanted tissues via EMB. It is envisioned that the system disclosed herein may be customizable with respect to the level of automation, i.e. the number and scope of components herein disclosed are performed automatically at the direction of the SHCU 14. At the opposite end of the spectrum from a fully automated system, SHCU 14 may operate software to guide a physician or other operator through a video monitor, audio cues, or some other means, through the steps of the procedure based on the software's determination of the best treatment protocol, such as by directing an operator where to place the EMB treatment probe 20, etc. As examples of semi-automation, SHCU 14 may be operatively connected to at least one robotic arm comprising an alignment tool capable of supporting probe 20, or providing an axis for alignment of probe 20, such that the tip of probe 20 is positioned at the correct point and angle at the surface of the patient's skin to provide a direct path along the longitudinal axis of probe 20 to the preferred location of the tip of probe 20 within the treatment area. In another embodiment, as described in more detail below, SHCU 14 provides audio or visual cues to the operator to indicate whether the insertion path of probe 20 is correct. In each of these variations and embodiments, the system, at the direction of SHCU 14, directs the planning, validation and verification of the Predicted Ablation Zone (to be described in more detail below), to control the application of therapeutic energy to the selected region so as to assure proper treatment, to prevent damage to sensitive structures, to enhance the patient's immunologic response to his cancer and/or to provide tracking, storage, transmission and/of retrieval of data describing the treatment applied.

In a preferred embodiment, SHCU is a data processing system comprising at least one application server and at least one workstation comprising a monitor capable of displaying to the operator a still or video image, and at least one input device through which the operator may provide inputs to the system, i.e. via a keyboard/mouse or touch screen, which runs software programmed to control the system in three "modes" of operation, wherein each mode comprises instructions to direct the system to perform one or more novel features of the present invention. The software of an embodiment of the present invention may preferably be operated from a personal computer connected to SHCU 14 via a direct, hardwire connection or via a communications network, such, that remote operation of the system is possible. The three contemplated modes are Biopsy mode, Planning Mode and Treatment Mode. However, it will be understood to one of ordinary skill in the art that the software and/or operating system may be designed differently while still achieving the same purposes. In all modes, the software can create, manipulate, and display to the user via a video monitor accurate, real-time three-dimensional images of the human body, which images can be zoomed, enlarged, rotated, animated, marked, segmented and referenced by the operator via the system's data input device(s). As described above, in various embodiments of the present invention the software and SHCU 14 can partially or fully control various attached components, probes, needles or devices to automate various functions of such components, probes, needles or devices, or facilitate robotic or remote control thereof.

### Biopsy Mode

The SHCU is preferably operatively connected to one or more external imaging sources such as an magnetic resonance imaging (MRI), ultrasound (US), electrical impedance tomography (EIT), or any other imaging device known in the art and capable of creating images of the human, body. Using inputs from these external sources, the SHCU first creates one or more "3D Fused Images" of the patient's body in the region of the detected cancer, suspected neoplasia, or unwanted tissue. External sources may include imaging of the lumen of the patient's bodily structure to locate suspicious images that may be neoplasia, in cases where the system is used to treat such maladies. The 3D Fused Images provide a 3D map of the selected treatment area within the patient's body over which locational data obtained from the one or more probes or needles according to embodiments of the present invention may be overlaid to allow the operator to monitor the treatment in real-time against a visual of the actual treatment area. Preferably, after the creation of a 3D Fused Image, a biopsy of the imaged area is taken (either immediately or at the convenience of the physician/patient) or the suspicion of tumor is confirmed by typical imaging characteristics.

In a first embodiment, a 3D Fused Image would be created from one or more MRI and ultrasound image(s) of the same area of the patient's body. An MRI image used for this purpose may comprise a multi-parametric magnetic resonance image created using, i.e., a 3.0 Telsa MRI scanner (such as Achieva, manufactured by Philips Healthcare) with a 16-channel cardiac surface coil (such as a SENSE coil, manufactured by Philips Healthcare) placed on the patient so as to support imaging of the area of concern in the patient. For example, for the treatment of prostate cancer, the surface coil may be placed over the pelvis of the patient with an endorectal coil (such as the BPX-30, manufactured by Medrad). For the treatment of sarcoma, MRI sequences obtained by this method preferably include: a tri-planar T2-weighted image, axial diffusion weighted imaging with apparent diffusion coefficient (ADC) mapping, 3-dimensional point resolved spatially localized spectroscopy, and an axial dynamic contrast enhanced MRI, An ultrasound image used for this purpose may be one or more 2D images obtained from one the use of equipment known in the art, including but not limited to: a standard biplane transrectal ultrasound probe (such as the Hitachi EUB 350), a standard biplane ultrasound transducer (such as the Hi Vision Preirus by Hitachi Aloka Medical America, Inc.), an endoscopic transducer such as an Olympus Curved Linear Array (GP-UC140P-AL5) connected to a ProSound F75 premium Hitachi Aloka, Ltd. ultrasound platform. The ultrasound image may be formed by, i.e., placing an EM field generator (such, as that manufactured by Northern Digital Inc.) on the patient, which allows for real-time tracking of a custom ultrasound probe embedded with a passive EM tracking sensor (such as that manufactured by Traxtal, Inc.).

In some embodiments, the US and guidance can be carried out with the commercially available EPIQ 7 G1 Ultrasound System, such as in the treatment of sarcoma or soft tissue tumors.

In one embodiment, the 3D fused image is then formed by the software according to the present invention by encoding the ultrasound data using position encoded data correlated to the resultant image by its fixed position to the US probe and/or transducer by the US scanning device. In an alternative embodiment, specifically for the treatment of prostate cancer, the 3D fused image is formed by encoding the ultrasound data using a position encoded prostate ultrasound stepping device (such as that manufactured by Civeo Inc) and then overlaying a virtual brachytherapy grid over the 3D ultrasound fused MM image. A brachytherapy grid is positionally correlated to the resultant image by its fixed position to the US probe by the US stepping device. Thus, in some embodiments, biopsy needle 200 does not need a locational sensor 26 because the positional guidance is provided by the brachytherapy grid. The software according to an embodiment of the present invention also records of the position of the obtained biopsy for later use in guiding therapy.

This protocol thus generates a baseline, diagnostic 3D Fused Image and displays the diagnostic 3D Fused Image to the operator in real time via the SHCU video monitor. Preferably, the system may request and/or receive additional 3D ultrasound images of the treatment area during treatment and fuse those subsequent images with the baseline 3D Fused image for display to the operator.

As an alternate means of creating the 3D Fused Image, a 2-dimensional sweep of the treatment area is performed in the axial plane to render a three-dimensional ultrasound image that is then registered and fused to a pre-biopsy MRI using landmarks common to both the ultrasound image and MRI image such as, for the treatment of prostate cancer, the capsular margins of the prostate and urethra. Where prostate cancer is the target, the sweep may be performed by a transrectal ultrasonography (TRUS) device. Lesions suspicions for cancer identified on MRI are semi-automatically superimposed on the real-time US (or TRUS) image. A biopsy device (such as that manufactured by Bard, Inc.) and embedded with a passive EM tracking device, as previously described, can then be tracked in relation to the position any areas of concern and tints a biopsy performed or, in alternative embodiments, an intraluminal biopsy taken using a biopsy device (such as an Olympus EZ Shot 2 Aspiration Needle) placed through the catheter of the EMB probe 20.

in yet another embodiment, and specifically for the treatment of prostate cancer, the 3D Fused Image may be created by placing the patient in the dorsal lithotomy position, placing a biopsy grid on the perineum, inserting a TRUS probe into the rectum and placing the transducer in the proper position prior to 3D data acquisition at the lateral margin of the prostate. The operator then activates the ultrasound probe to capture multiple images. The computer then reconstructs a 3D image of the prostate by displaying the image in a multi-planer reformation (MPR) mode and displays grid lines through the 3D volume that correspond to the holes in the grid on the patient's perineum. At this point, the reconstructed MRI data can be fused to the ultrasound date using the previously described methods. Such a system was described in Onik GM, Downey DB, Fenster A, Sonographically Monitoring Cryosurgery In A Prostate Phantom, journal of Ultrasound 16:2:67-270 (1996).

The 3D Fused Image as created by any one of the above methods is then stored in the non-transitive memory of the SHCU, which may employ additional software to locate and electronically tag within the 3D Fused Image specific areas in the treatment area or its vicinity, including sensitive or critical structures and areas that require anesthesia such as, for example, the Neurovascular Bundles (for the treatment of prostate cancer), i.e. to enable the guidance of standard or trackable anesthesia needles to those locations. The SHCU then displays the 3D Fused image to the operator alone or overlaid with locational data from each of the additional devices described herein where available. The 3D Fused Image may be presented in real time in sector view, or the software may be programmed to provide other views based on design preference. As described above, the software may then direct the operator and/or a robotic arm to take a biopsy of the identified area of cancerous tissue or in a specific location of concern based on an analysis of the imaging data and record the results of same, which biopsy may be tracked in real time. Analysis of the biopsy tissue, which may be done by the system or a physician/technician, will indicate whether the biopsied tissue is cancerous. Thus, a 3D map of cancerous tissue in the area of concern within the patient's body may be created in this way. The software may employ an algorithm to determine where individual biopsies should be taken based on optimal spacing between same or based on the location of other biopsies that revealed cancerous tissue to ensure that all areas of cancerous- tissue, in. the region have been located and indexed against the 3D Fused Image.

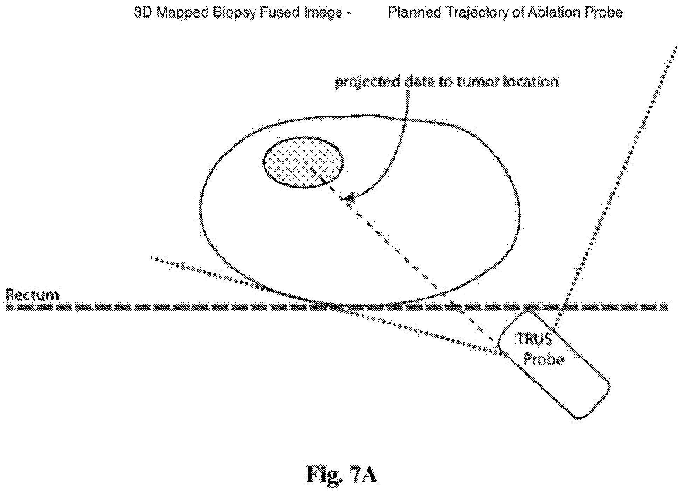

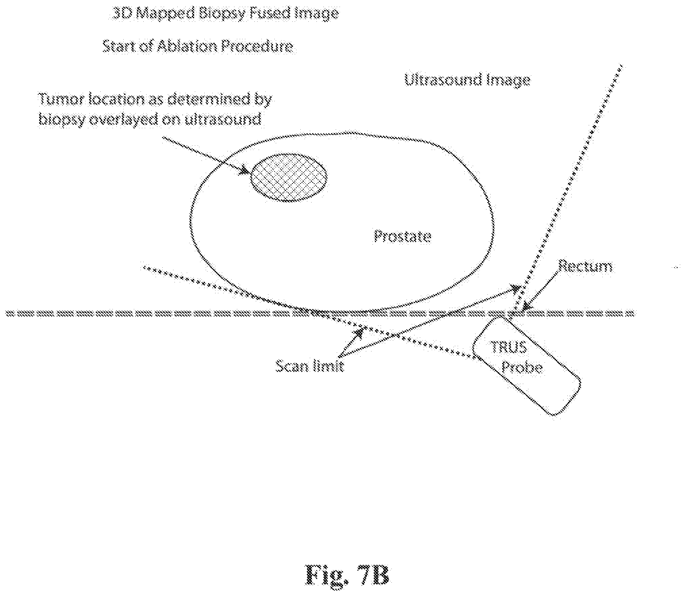

Using the biopsy result data in conjunction with the 3D fused Image, the software can create a "3D Mapped Biopsy Fused Image", which can be used as the basis for planning an office based or in-patient treatment procedure for the patient (see FIGs. 7A-7B). The SHCU also preferably stores the biopsy sample information indexed to sample location, orientation and number, which information can be provided to a pathologist or other treatment provider via a communications network to be displayed on his or her remote workstation, allowing the other treatment provider to interact with and record pathological findings about each sample in real time.

### Planning Mode

Upon generation of one or more 3D Fused Images of the planned treatment area and, preferably completion of one or more biopsies of the affected area, the SHCU may display to the operator via a video terminal the precise location(s) of one or more areas in the prostate (or other treatment area), or its vicinity, which require therapy, via annotations or markers on the 3D Fused Image(s): this area requiring therapy is termed the Target Treatment Zone. This information is then used by the system or by a physician to determine optimal placement of the EMB treatment probe(s) 20, Importantly, the 3D Fused Image should also contain indicia to mark Neurovascular Bundles (NVB), where present, or other anesthesia targets designated by the physician, the location of which will be used to calculate a path for placement of one or more anesthesia needles for delivery of local anesthesia to the treatment area. If necessary due to changes in gland, tumor or tissue size, the geographic location of each marker can be revised and repositioned, and the 3D Fused Image updated in real time by the software, using 3D ultrasound data as described above. The system may employ an algorithm for detecting changes in gland, tumor or tissue size and requesting additional ultrasound scans, may request ultrasound scans on a regular basis, or the like.

In a preferred embodiment, the software may provide one or more "virtual" EMB treatment probes 20 (of the various types described above) which may be overlaid onto the 3D Fused Image by the software or by the treatment provider to determine the extent of ablation that would be accomplished with each configuration. Where a non-catheter-type probe is used, the virtual probes also define a path to the target point by extending a line or path from the target point to a second point defining the entry point on the skin surface of the patient for insertion of the real EMB treatment probe. Preferably, the software is configured to test several possible probe 20 placements and calculate the probable results of treatment to the affected area via such a probe 20 (the Predicted Ablation Zone) placement using a database of known outcomes from various EMB treatment protocols or by utilizing an algorithm which receives as inputs various treatment parameters such as pulse number, amplitude, pulse width and frequency. By comparing the outcomes of these possible probe locations to the tumor volume as indicated by the 3D Fused linage and/or the 3D Mapped Biopsy Fused Image, the system may determine the optimal probe 20 placement. Alternatively, the system may be configured to receive inputs from a physician to allow him or her to manually arrange and adjust the virtual EMB treatment probes to adequately cover the treatment area and volume based on his or her expertise. The system may utilize virtual anesthesia needles in the same way to plan treatment.

When the physician is satisfied with the Predicted Ablation Zone coverage shown on the Target Treatment Zone based on the placement and configuration of the virtual EMB treatment, probes and the virtual anesthesia, needles, as- determined by the system of by the physician himself, the physician "confirms" in the system (i.e. "locks in") the three-dimensional placement and energy/medication delivery configuration of the grouping of virtual EMB treatment probes and virtual anesthesia needles, and the system registers the position of each as an actual software target to be overlaid on the 3D Fused Image and used by the system for guiding the insertion or placement of the real probe(s) and needle(s) according to the present invention (which may be done automatically by the system via robotic arms or by the physician by tracking his or her progress on the 3D Fused Image.

If necessary, EMB treatment, as described in further detail below, may be carried out immediately after a biopsy of the patient is performed. Alternately, EMB treatment may take place days or even weeks after one or more biopsies are performed. In the latter case, the steps described with respect to the Planning Mode, above, may be undertaken by the software/physician at any point between biopsy(s) and treatment.

### Treatment Mode

The software displays, via the SHCU video monitor, the previously confirmed and "locked in" Target Treatment Zone, Predicted. Ablation Zone and 3D Mapped Biopsy Fused image, with the location and configuration of all previously confirmed virtual probes/needles and their calculated insertion points, angular 3D geometry, and insertion depths or placement when inserted intraluminally, which can be updated as needed at time of treatment to reflect any required changes as described above.

Using the planned locations and targets established for the delivery of anesthesia, and the displayed insertions paths, the software then guides the physician (or robotic arm) in real time to place one or more anesthesia needles and then to deliver the appropriate amount of anesthesia to the targeted locations (i.e., in the vicinity of the Neurovascular Bundles). Deviations from the insertion path previously determined by the system in relation to the virtual or placement location of the needles/probes may be highlighted by the software in real time so as to allow correction of targeting at the earliest possible time in the process. This same process allows the planning and placement of local anesthesia needles as previously described. In some embodiments, the system may employ an algorithm to calculate the required amount of anesthesia based on inputs such as the mass of the tissue to be treated and individual characteristics of the patient which may be inputted to the system manually by the operator or obtained from a central patient database via a communications network, etc.

Once anesthesia has been administered, the system displays the Predicted Ablation Zone and the boundaries thereof as an overlay on the 3D Fused Image including the Target Treatment Zone and 3D Mapped Biopsy Fused Image and directs the physician (or robotic arm) as to the placement of each EMB treatment probe 20. The Predicted Ablation Zone may be updated and displayed in real time as the physician positions each probe 20 to give graphic verification of the boundaries of the Target Treatment Zone, allowing the physician to adjust and readjust the positioning of the Therapeutic EMB Probes, sheaths, electrode exposure and other treatment parameters (which in turn are used to update the Predicted Ablation Zone). When the physician (or, in the case of a fully automated system, the software) is confident of accurate placement of the probes, he or she may provide such an input to the system, which then directs the administration of EMB pulses via the EMB pulse generator 16 and probes 20.

The SHCU controls the pulse amplitude 30, frequency 31, polarity and shape provided by the EMB pulse generator 16, as, well as the number of pulses 32 to be applied in the treatment series or pulse train, the duration of each pulse 32, and the inter pulse burst delay 33. Although only two are depicted in FIG. 10 due to space constraints, EMB ablation is preferably performed by application of a series of not less than 100 electric pulses 32 in a pulse train so as to impart the energy necessary on the target tissue 2 without developing thermal issues in any clinically significant way. The width of each individual pulse 32 is preferably from 100 to 1000 µs with an inter pulse hurst interval 33 during which no voltage is applied in order to facilitate heat dissipation and avoid thermal effects. The relationship between the duration of each pulse 32 and the frequency 31 (period) determines the number of instantaneous charge reversals experienced by the cell membrane during each pulse 32, The duration of each inter pulse burst interval 33 is determined by the controller 14 based on thermal considerations. In an alternate embodiment the system is further provided with a temperature probe 22 inserted proximal to the target tissue 2 to provide a localized temperature reading at the treatment site to the SHCU 14. The temperature probe 22 may be a separate, needle type probe having a thermocouple tip, or may be integrally formed with or deployed from one or more of the needle electrodes, or the Therapeutic EMB Probes. The system may further employ an algorithm to determine proper placement of this probe for accurate readings from same. With temperature feedback in real time, the system can modulate treatment parameters to eliminate thermal effects as desired by comparing the observed temperature with various temperature set points stored in memory. More specifically, the system can shorten or increase the duration of each pulse 32 to maintain a set temperature at the treatment site to, for example, create a heating (high temp) for the needle tract to prevent bleeding or to limit heating (low temp) to prevent any coagulative necrosis. The duration of the inter pulse burst interval can be modulated in the same manner in order to eliminate the need to stop treatment and maximizing the deposition of energy to. accomplish EMB. Pulse amplitude 30 and total number of pulses in the pulse train may also be modulated for the same purpose and result.

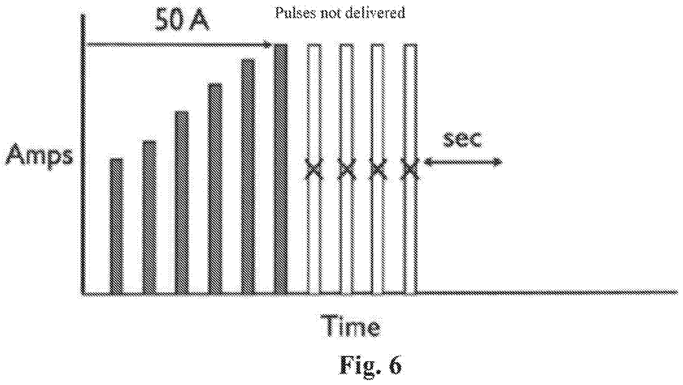

In yet another embodiment, the SHCU may monitor or determine current flow through the tissue during treatment for the purpose of avoiding overheating while yet permitting treatment to continue by reducing the applied voltage. Reduction in tissue impedance during treatment due to charge buildup and membrane rupture can cause increased current flow which engenders additional heating at the treatment site. With reference to FIG. 6, prior treatment methods have suffered from a need to cease treatment when the current exceeds a maximum allowable such that treatment goals are not met. As with direct temperature monitoring, embodiments of the present invention can avoid the need to stop treatment by reducing the applied voltage and thus current through the tissue to control and prevent undesirable clinically significant thermal effects. Modulation of pulse duration and pulse burst interval duration may also be employed by the controller 14 for this purpose as described.

During treatment, the software captures all of the treatment parameters, all of the tracking data and representational data in the Predicted Ablation Zone, the Target Treatment Zone and in the 3D Mapped Biopsy Fused Image as updated in real time to the moment of therapeutic trigger. Based on the data received by the system during treatment, the treatment protocol may be adjusted or repeated as necessary.

The software may also store, transmit and/or forwarding treatment data to a central database located on premises in the physician's office and/or externally via a communications network so as to facilitate the permanent archiving and retrieval of all procedure related data. This will facilitate the use and review of treatment data, including for diagnostic purposes and pathology related issues, for treatment review purposes and other proper legal purposes including regulatory review.

The software may also transmit treatment data in real time to a remote proctor/trainer who can interact in real time with the treating physician and all of the images displayed on the screen, so as to insure a safe learning experience for an inexperienced treating physician, and so as to archive data useful to the training process and so as to provide system generated guidance for the treating physician. In another embodiment, the remote proctor can control robotically all functions of. the system.

Optionally, with one or more EMB treatment probes 20 still in place within the ablated tissue, the physician or system can perform injection of immunologic adjuvant agents, or other materials into the ablated tissue, using capabilities built into the probe, as described above, or through separate delivery means.

In other embodiments of the present invention, some or all of the treatment protocol may be completed by robotic arms, which may include an ablation probe guide which places the specially designed Therapeutic EMB Probe (or an ordinary ablation probe but with limitations imposed by its design) in the correct trajectory or intraluminal location relative to the tumor. Robotic arms may also be used to hold the US transducer in place and rotate it to capture images for a 3D US reconstruction. Robotic arms can be attached to an anesthesia needle guide which places the anesthesia needle in the correct trajectory to the targeted anesthesia areas to guide the delivery of anesthesia by the physician.

In other embodiments, the robotic arm can hold the anesthesia needle itself or a trackable anesthesia needle (see FIG. 1.4) with sensor-transmitters and actuators built in, that can be tracked in real time, and that can feed data to the software to assure accurate placement thereof and enable the safe, accurate and effective delivery of anesthesia to the targeted anesthesia areas and other regions, and can directly insert the needle into the targeted areas of the Neurovascular Bundle and other regions using and reacting robotically to real, time positioning data supported by the 3D Mapped Biopsy Fused Image and Predicted Ablation Zone data and thereby achieving full placement robotically, and upon activation of the flow actuators, the delivery of anesthesia as planned or confirmed by the physician.

In addition, the robotic arm can hold the Therapeutic EMB Probe itself and can directly insert the probe into the patient's tumor (or into an intraluminal location proximate the tumor) using and reacting robotically to real time positioning data supported by the 3D Mapped Biopsy Fused image and Predicted Ablation Zone data and thereby achieving full placement robotically.

Robotic components capable of being used for these purposes include the iSR'obotTM Mona Lisa robot, manufactured by Biobot Surgical Pte. Ltd. In such embodiments the Software supports industry standard robotic control and programming languages such as RAIL, AML, VAL, AL, RPL, PYRO, Robotic Toolbox for MATLAB and OPRoS as well as other robot manufacturer's proprietary languages.

In yet another embodiment, tissue characterization ability which is built into the EMB probe itself can identify the cancerous area and then allow direct destruction of the tumor in a one step procedure eliminating the need for the separate biopsy and pathological examination.

In yet another embodiment, the ablation is made to a metastatic lesion in an organ other than the primary cancer organ, using all the capabilities of the system outlined above. In the treatment of a metastatic lesion, the lesion may further be directly injected with immune enhancing drugs to facilitate a tumor specific immune response.

**In** another embodiment, the disease type treated is a squamous cell carcinoma or basal cell carcinoma. **In** yet another embodiment, the skin lesion treated is a benign lesion such as a neurofibroma. **In** yet another embodiment, the skin lesion treated is a lipoma located subcutaneously.

**In** other embodiments, the system as described above is used to treat prostate neoplasia or **BPH** from an intraurethral location. **In** yet other embodiments, the system is used to treat esophageal carcinoma or Barrels esophagus.

**In** yet another embodiment, the system with the intraluminal probe is used inside the bile duct, pancreatic duct or bowel to treat pancreatic carcinoma. **In** another embodiment, the system using the intraluminal probe is used to treat bile duct carcinoma from an intraluminal location inside the bile duct.

The SHCU can fully support Interactive Automated Robotic Control through a proprietary process for image sub-segmentation of the treatment area and nearby anatomical structures for planning and performing robotically guided biopsy and therapeutic interventions in an office based or in-patient setting.

Sub-segmentation is the process of capturing and storing precise image detail of the location size and placement geometry of the described anatomical object so as to be able to define, track, manipulate and display the object and particularly its three-dimensional boundaries and accurate location in the body relative to the rest, of the objects in the field and to the anatomical registration of the patient in the system so as to enable accurate three-dimensional targeting of the object or any part thereof, as well as the three-dimensional location of its boundaries in relation to the locations of all other sub segmented objects and computed software targets and needle and probe pathways. The software sob-segments out various critical, substructures in or proximate to the treatment area, such as the neuro-vascular bundles, peripheral zone, ejaculatory ducts, urethra, rectum, and Denonvilliers Fascia in a systematic and programmatically supported and required fashion, which is purposefully designed to provide and enable the component capabilities of the software as described herein.

Having now fully set forth the preferred embodiment and certain modifications of the concept underlying the present invention, various other embodiments as well as certain variations and modifications of the embodiments herein shown and described will obviously occur to those skilled in the art upon becoming familiar with said underlying concept. It is to be understood, therefore, that the invention may be practiced otherwise than as specifically set forth herein.

The invention is defined by the following claims.

### STATEMENT OF INDUSTRIAL APPLICABILITY

The term "cancer" refers to a group of diseases characterized by sustained cell proliferation, reduced or delayed cell mortality, cooption of bodily angiogenesis and metabolic processes and evasion of bodily immune response which results in undesirable soft tissue growths or tumors which often result in the death of the affected person. Specific types of cancer or unwanted tissue such as prostate cancer, unresectable pancreatic cancer, tumors of the breast or soft tissue, melanoma, ductal carcinoma, neoplasia, or intra and extra luminal abnormal tissue have low survival rates in humans, and the known treatments for same are often painful, debilitating, and/or ineffective. There would be great industrial applicability in an effective ablation of any of the above types of cancerous cells or other unwanted tissue that was minimally invasive and less traumatic than classic methods of removing cancerous or unwanted tissue by surgical excision, and which could be conducted without the need for general anesthesia, which may have dangerous side effects. The instant invention fulfills this need by utilizing Radio-Frequency Electrical Membrane Breakdown to destroy the cellular membranes of unwanted or cancerous tissue without denaturing the intra-cellular contents of the cells comprising the tissue, thereby exposing tumor antigens and other infra-cellular components which can have an immunologic effect on local or distant cancerous tissue. In addition, the instant invention may be used with or without the addition of immunologic adjuvant drugs, agents, or materials to further improve the chances of successful total removal of cancerous cells.

## Claims

1. A system for ablating undesirable soft tissue in a living subject using radio frequency electrical membrane breakdown (EMB), the system comprising:
at least one EMB pulse generator (16) capable of producing a high voltage bi-polar pulse to generate an electric field in the range of 1,500 V/cm to 10,000 V/cm, the electric field being sufficient to cause electrical membrane breakdown of a cell membrane of a plurality of cells of said soft tissue to cause immediate spillage of all intracellular components into an extracellular space and exposure of an internal constituent part of said cell membrane to said extracellular space;
at least one EMB treatment probe (20) capable of delivering said electric field to said soft tissue the EMB treatment probe comprising a core (21) comprising an electrically conductive material, and an outer electrode (24) on at least one side of the core; and
at least one software hardware control unit, (SHCU), operatively connected to said at least one EMB pulse generator and said at least one EMB treatment probe.

2. The system of claim 1, wherein said at least one EMB treatment probe further comprises:
an insulating sheath (23) comprised of a non-electrically-conductive material, said insulating sheath forming a barrier between the core and said outer electrode.

3. The system of claim 2, wherein said outer electrode (24) is mounted on said insulating sheath (23), and wherein said outer electrode and said insulating sheath are movable as a unit along a lateral dimension of said core to enable adjustment of the lateral distance between a distal end of said core and said outer electrode.

4. The system of claim 3, further comprising at least one electromagnetic sensor (26) on each of said core (21) and said outer electrode (24).

5. The system of claim 2, wherein said at least one EMB treatment probe comprises at least one sensor (26) capable of determining or quantifying cell death in tissue adjacent to said at least one sensor.

6. The system of claim 2, wherein said at least one EMB treatment probe comprises a hollow interior defined by an inner lumen of sufficient diameter to accommodate one or more surgical tools or devices of one or more standard gauges or sizes.

7. The system of claim 6, wherein said at least one EMB treatment probe comprises an outer electrode on an outer surface thereof, and further comprising a needle sized to fit within said inner lumen of said EMB treatment probe, said needle comprising a needle electrode on a distal end thereof, wherein a polarity of said needle electrode is not equal to a polarity of said outer electrode.

8. The system of claim 2, wherein said at least one EMB treatment probe comprises an expandable balloon at a distal end thereof, said expandable balloon further comprising one or more electrodes for delivering said electric field.

9. The system of claim 2, wherein said at least one EMB treatment probe is a catheter-type probe, wherein said at least one EMB treatment probe further comprises:
a central lumen;
a positive electrode disposed at a first location on an outer surface of said EMB treatment probe; and
a negative electrode disposed on at a second location on an outer surface of said EMB treatment probe, said first location and said second location being separated along a longitudinal dimension of said at least one EMB treatment probe.

10. The system of claim 9, wherein one of said positive electrode or said negative electrode is disposed on the end of an insulating sheath comprised of a non-electrically-conductive material, said insulating sheath being movable along a longitudinal axis of said at least one EMB treatment probe.

11. The system of claim 2, wherein said at least one EMB treatment probe further comprises one or more stents.

12. The system of claim 11, wherein said one or more stents each comprise conducting and non-conducting areas corresponding to said at least one electrode.

13. The system of claim 1, further comprising at least one trackable biopsy needle.

14. The system of claim 1, wherein said at least one EMB treatment probe is rigid, semirigid, semi-flexible or flexible.

15. The system of claim 1, wherein at least a portion of said system is robotically controlled, and wherein said electric field is sufficient to cause electrical membrane breakdown of a cell membrane of a plurality of cells of said soft tissue to cause immediate spillage of at least one antigen into an extracellular space.

## Patentansprüche

1. System zum Abtragen von unerwünschtem Weichgewebe in einem lebenden Subjekt unter Verwendung von hochfrequentem elektrischem Membrandurchbruch (EMB), wobei das System umfasst:
mindestens einen EMB-Impulsgenerator (16), der in der Lage ist, einen bipolaren Hochspannungsimpuls zu erzeugen, um ein elektrisches Feld in dem Bereich von 1.500 V/cm bis 10.000 V/cm zu erzeugen, wobei das elektrische Feld ausreichend ist, um einen elektrischen Membrandurchbruch einer Zellmembran einer Mehrzahl von Zellen des Weichgewebes zu bewirken, um einen unmittelbaren Austritt aller intrazellulären Komponenten in einen extrazellulären Raum und eine Exposition eines inneren Bestandteils der Zellmembran gegenüber dem extrazellulären Raum zu bewirken;
mindestens eine EMB-Behandlungssonde (20), die dazu in der Lage ist, das elektrische Feld an das Weichgewebe abzugeben, wobei die EMB-Behandlungssonde einen Kern (21), der ein elektrisch leitfähiges Material umfasst, und eine Außenelektrode (24) auf mindestens einer Seite des Kerns umfasst; und
mindestens eine Software-Hardware-Steuereinheit (SHCU), die mit dem mindestens einen EMB-Impulsgenerator und der mindestens einen EMB-Behandlungssonde operativ verbunden ist.

2. System nach Anspruch 1, wobei die mindestens eine EMB-Behandlungssonde ferner umfasst:
eine isolierende Hülle (23), die aus einem nicht elektrisch leitfähigen Material besteht, wobei die isolierende Hülle eine Barriere zwischen dem Kern und der Außenelektrode bildet.

3. System nach Anspruch 2, wobei die Außenelektrode (24) an der isolierenden Hülle (23) montiert ist und wobei die Außenelektrode und die isolierende Hülle als eine Einheit entlang einer lateralen Abmessung des Kerns bewegbar sind, um eine Einstellung des lateralen Abstands zwischen einem distalen Ende des Kerns und der Außenelektrode zu ermöglichen.

4. System nach Anspruch 3, ferner umfassend mindestens einen elektromagnetischen Sensor (26) an jedem des Kerns (21) und der äußeren Elektrode (24).

5. System nach Anspruch 2, wobei die mindestens eine EMB-Behandlungssonde mindestens einen Sensor (26) umfasst, der in der Lage ist, Zelltod im Gewebe neben dem mindestens einen Sensor zu bestimmen oder zu quantifizieren.

6. System nach Anspruch 2, wobei die mindestens eine EMB-Behandlungssonde einen hohlen Innenraum umfasst, der durch ein Innenlumen mit ausreichendem Durchmesser definiert ist, um ein oder mehrere chirurgische Werkzeuge oder Vorrichtungen eines oder mehrerer Standardkaliber oder -größen aufzunehmen.

7. System nach Anspruch 6, wobei die mindestens eine EMB-Behandlungssonde eine Außenelektrode an einer Außenfläche davon umfasst und ferner eine Nadel umfasst, die dazu bemessen ist, in das Innenlumen der EMB-Behandlungssonde zu passen, wobei die Nadel eine Nadelelektrode an einem distalen Ende davon umfasst, wobei eine Polarität der Nadelelektrode nicht gleich einer Polarität der Außenelektrode ist.

8. System nach Anspruch 2, wobei die mindestens eine EMB-Behandlungssonde einen expandierbaren Ballon an einem distalen Ende davon umfasst, wobei der expandierbare Ballon ferner eine oder mehrere Elektroden zum Bereitstellen des elektrischen Feldes umfasst.

9. System nach Anspruch 2, wobei die mindestens eine EMB-Behandlungssonde eine katheterartige Sonde ist, wobei die mindestens eine EMB-Behandlungssonde ferner umfasst:
ein mittiges Lumen;
eine positive Elektrode, die an einer ersten Stelle auf einer Außenfläche der EMB-Behandlungssonde angeordnet ist; und
eine negative Elektrode, die an einer zweiten Stelle auf einer Außenfläche der EMB-Behandlungssonde angeordnet ist, wobei die erste Stelle und die zweite Stelle entlang einer Längsabmessung der mindestens einen EMB-Behandlungssonde getrennt sind.

10. System nach Anspruch 9, wobei eine der positiven Elektrode oder der negativen Elektrode an dem Ende einer Isolierhülle angeordnet ist, die aus einem nicht elektrisch leitfähigen Material besteht, wobei die Isolierhülle entlang einer Längsachse der mindestens einen EMB-Behandlungssonde beweglich ist.

11. System nach Anspruch 2, wobei die mindestens eine EMB-Behandlungssonde ferner einen oder mehrere Stents umfasst.

12. System nach Anspruch 11, wobei der eine oder die mehreren Stents jeweils leitende und nicht leitende Bereiche umfassen, die der mindestens einen Elektrode entsprechen.

13. System nach Anspruch 1, ferner umfassend mindestens eine verfolgbare Biopsienadel.

14. System nach Anspruch 1, wobei die mindestens eine EMB-Behandlungssonde starr, halbstarr, halbflexibel oder flexibel ist.

15. System nach Anspruch 1, wobei mindestens ein Teil des Systems robotisch gesteuert wird und wobei das elektrische Feld ausreichend ist, um einen elektrischen Membrandurchbruch einer Zellmembran einer Mehrzahl von Zellen des Weichgewebes zu bewirken, um ein unmittelbares Austreten mindestens eines Antigens in einen extrazellulären Raum zu bewirken.

## Revendications

1. Système pour l'ablation de tissu mou indésirable chez un sujet vivant en utilisant une rupture de membrane électrique (RME) par radiofréquence, le système comprenant :
au moins un générateur d'impulsions de RME (16) capable de produire une impulsion bipolaire à haute tension pour générer un champ électrique dans la plage de 1,500 V/cm à 10,000 V/cm, le champ électrique étant suffisant pour provoquer une ablation tissulaire ciblée d'une pluralité de cellules dudit tissu mou afin d'entraîner un déversement immédiat de tous les composants intracellulaires dans un espace extracellulaire et l'exposition d'une partie constituante interne de ladite membrane cellulaire audit espace extracellulaire ;
au moins une sonde de traitement de RME (20) capable d'administrer ledit champ électrique audit tissu mou, la sonde de traitement de RME comprenant un noyau (21) comprenant un matériau électriquement conducteur, et une électrode externe (24) sur au moins un côté du noyau ; et
au moins une unité de commande logicielle et matérielle, (UCLM), connectée de manière opérationnelle audit au moins un générateur d'impulsions de RME et à ladite au moins une sonde de traitement de RME.

2. Système selon la revendication 1, dans lequel ladite au moins une sonde de traitement de RME comprend en outre :
une gaine isolante (23) constituée d'un matériau non électriquement conducteur, ladite gaine isolante formant une barrière entre le noyau et ladite électrode externe.

3. Système selon la revendication 2, dans lequel ladite électrode externe (24) est montée sur ladite gaine isolante (23), et dans lequel ladite électrode externe et ladite gaine isolante sont mobiles d'un seul bloc le long d'une dimension latérale dudit noyau pour permettre un ajustement de la distance latérale entre une extrémité distale dudit noyau et ladite électrode externe.

4. Système selon la revendication 3, comprenant en outre au menos un capteur électromagnétique (26) sur chacun dudit noyau (21) et de ladite électrode externe (24).

5. Système selon la revendication 2, dans lequel ladite au moins une sonde de traitement de RME comprend au moins un capteur (26) capable de surveiller l'état des tissus adjacents audits au moins un capteur.

6. Système selon la revendication 2, dans lequel ladite au moins une sonde de traitement de RME comprend un intérieur creux défini par une lumière interne d'un diamètre suffisant pour loger un ou plusieurs outils ou dispositifs chirurgicaux de dimensions standard.

7. Système selon la revendication 6, dans lequel ladite au moins une sonde de traitement de RME comprend une électrode externe sur une surface externe de celle-ci, et comprenant en outre une aiguille dimensionnée pour s'adapter à l'intérieur de ladite lumière interne de ladite sonde de traitement de RME, ladite aiguille comprenant une électrode d'aiguille sur une extrémité distale de celle-ci, dans lequel une polarité de ladite électrode d'aiguille n'est pas égale à une polarité de ladite électrode externe.

8. Système selon la revendication 2, dans lequel ladite au moins une sonde de traitement de RME comprend un ballonnet expansible au niveau d'une extrémité distale de celle-ci, ledit ballonnet expansible comprenant en outre une ou plusieurs électrodes pour administrer ledit champ électrique.

9. Système selon la revendication 2, dans lequel ladite au moins une sonde de traitement de RME est une sonde de type cathéter, dans lequel ladite au moins une sonde de traitement de RME comprend en outre :
une lumière centrale ;
une électrode positive disposée au niveau d'un premier emplacement sur une surface externe de ladite sonde de traitement de RME ; et
une électrode négative disposée au niveau d'un second emplacement sur une surface externe de ladite sonde de traitement de RME, ledit premier emplacement et ledit second emplacement étant séparés le long d'une dimension longitudinale de ladite au moins une sonde de traitement de RME.

10. Système selon la revendication 9, dans lequel l'une de ladite électrode positive ou de ladite électrode négative est disposée sur l'extrémité d'une gaine isolante constituée d'un matériau non électriquement conducteur, ladite gaine isolante étant mobile le long d'un axe longitudinal de ladite au moins une sonde de traitement de RME.

11. Système selon la revendication 2, dans lequel ladite au moins une sonde de traitement de RME comprend en outre un ou plusieurs stents.

12. Système selon la revendication 11, dans lequel lesdits un ou plusieurs stents comprennent chacun des zones conductrices et non conductrices correspondant à ladite au moins une électrode.

13. Système selon la revendication 1, comprenant en outre au moins une aiguille de biopsie traçable.

14. Système selon la revendication 1, dans lequel ladite au moins une sonde de traitement de RME est rigide, semi-rigide, semi-flexible ou flexible.

15. Système selon la revendication 1, dans lequel au moins une partie dudit système est commandée par robot, et dans lequel ledit champ électrique est configuré pour induire une réponse immunogène par l'exposition de composants cellulaires du tissu mou dans un espace extracellulaire.
